# EUROPEAN PATENT APPLICATION

(11) **EP 2 410 341 A1**
(43) Date of publication of application: **25.01.2012**
(21) Application number: 10753481.0
(22) Date of filing: 15.03.2010
(51) Int. Cl.: G01N 35/02, C12M 1/00, C12N 15/09, G01N 37/00, G01N 33/53

(54) **ANALYSIS CHIP, ANALYSIS METHOD AND METHOD FOR STIRRING SOLUTION**

(30) Priority: 16.03.2009 JP 2009063344
(71) Applicant: Toray Industries, Inc., Tokyo 103-8666 (JP)
(72) Inventor: BABA, Tomo, Kamakura-shi Kanagawa 248-8555 (JP); TAKII, Yuki, Kamakura-shi Kanagawa 248-8555 (JP)
(74) Representative: Kador & Partner
(86) International application number: PCT/JP2010/054292
(87) International publication number: WO 2010/106989

(57) **Abstract**

[Object] The invention provides an analysis chip by which the efficiency of reaction with a selective binding substance is promoted by using particles having a large size to promote the efficiency of stirring of the test substance solution without damaging the portion of the carrier on which the selective binding substance is immobilized, by the moving particles when stirring the test substance solution with the particles.

[Means of Solution] An analysis chip including a carrier having a surface on which a selective binding substance(s) is(are) immobilized; a vessel holding a solution containing a test substance(s) which react(s) with the selective binding substance(s); and particles for stirring the solution, the particles being sealed within a space formed between the carrier and the vessel; wherein a separator which allows passage therethrough of the solution containing the test substance(s) but does not allow passage therethrough of the particles is arranged in the space so as to separate the surface of the carrier on which the selective binding substance(s) is(are) immobilized and the particles; a method of analysis using the analysis chip; and a method of stirring a solution using the analysis chip.

## Description

### TECHNICAL FIELD

The present invention relates to an analysis chip comprising a carrier on which a substance(s) (herein referred to as "selective binding substance") selectively binding to a test substance is(are) immobilized, which chip is for measuring the amount of a test substance bound to the carrier through the selective binding substance by selectively binding the solution containing the test substance with the selective binding substance, as well as to an analysis method and a method of stirring the solution using the analysis chip.

### BACKGROUND ART

Genetic information analyses of various organisms have been started. Information concerning a number of genes including human gene and base sequences thereof, proteins encoded by the gene sequences and sugar chains secondarily produced from the proteins have been rapidly revealed. The functions of macromolecules such as genes, proteins and sugar chains whose sequences have been clarified can be examined by various methods. As major ones, regarding nucleic acids, the relationships between various genes and expressions of biological functions thereof can be examined by utilizing the nucleic acid/nucleic acid complementarity of various nucleic acids by a method such as Northern blotting or Southern blotting. As for proteins, functions of proteins and expressions thereof can be examined by utilizing the protein/protein reactions by a method represented by Western blotting.

In recent years, as a method of analyzing expressions of a number of genes in one time, a novel analysis method called DNA microarray method (DNA chip method) has been developed and is drawing attention. This method is to detect and quantify nucleic acids based on nucleic acid/nucleic acid hybridization reaction, so that its principle is the same as those of the conventional methods. This DNA chip method can be applied to the detection and quantification of proteins or sugar chains based on the protein/protein, sugar chain/sugar chain, or sugar chain/protein specific reactions. This technique is largely **characterized in that** a flat glass substrate called microarray or DNA chip on which a number of DNA fragments, proteins or sugar chains are arrayed and immobilized at a high density is used. Examples of the concrete method of using the DNA chips include methods wherein genes as a sample expressed in a cell of interest labeled with a fluorescent dye or the like are subjected to hybridization on the flat substrate with nucleic acids (DNAs or RNAs) complementary thereto, and the sites at which the hybridization occurs are detected by high speed scanning using a high resolution detection apparatus (scanner); and methods wherein responses such as electric currents based on electrochemical reactions are detected. By these methods, the amount of the respective genes in the sample can be quickly estimated. Further, the application field of the DNA chips is not limited to the expression analyses of genes, but they are largely expected as means for detecting single nucleotide polymorphisms (SNPs) of genes.

DNA chips are now mostly used for examining a number of genes in one time for research purpose, using the chip on which the genes as many as several tens thousands to several thousands are mounted. In the future, it is expected that DNA chips will be used in the field of diagnoses. In case of using a DNA chip for diagnosis, the amount of the test substance which can be collected may be very small. In such a case, the sensitivity of the current DNA chips is not sufficient, so that the measurement of the test substance may be difficult. Moreover, with the current DNA chips, the fluorescence intensity after the hybridization of a gene whose expression level is small is very weak, so that such a gene may not be analyzed in practice. Thus, it is a problem of the current DNA chips to increase the fluorescence intensity after hybridization in cases where the amount of the test substance is small or the expression level of the gene is small. To solve this problem, a point is to how efficiently DNAs and probe DNAs are reacted. As a method of efficiently reacting DNAs which are test substances and the probes, since the natural diffusion of the test substances is insufficient, it has been proposed to stir the solution so as to accelerate the reaction between the probes and the test substances.

As an example where the test substance solution is stirred, Patent Document 1 discloses a method wherein a test substance solution mixed with particles or bubbles is brought into contact with a carrier on which selective binding substances reacting with the test substances are immobilized, and the test substance solution is stirred by moving the particles or bubbles to increase the efficiency of the reaction with the test substances, thereby increasing the signal intensities after the hybridization.

Patent Document 2 discloses a method wherein a plurality of subblock regions are formed in which protrusions on which selective binding substances are respectively immobilized are arrayed in the form of matrix, and the test substance solution is stirred by moving particles or microrods in the recesses in the subblock regions and in the recesses between the subblock regions, thereby increasing the reaction efficiency with the test substances. The width of the recesses between the subblock regions is larger than that of the recesses in the subblock regions, and the particles or the microrods are preliminarily housed.

Further, Patent Document 3 and Patent Document 4 disclose a method wherein the test substance solution is stirred by moving magnetic beads in the test substance solution by magnetic force, so as to increase the efficiency of the reaction with the test substance. Patent Document 4 discloses a method wherein a test substance solution in which beads are mixed is brought into contact with the DNA chip, the solution is sealed with a cover glass or the like, and the chip is rotated to drop the beads in the direction of gravity so as to stir the test substance solution, thereby increasing the signal after the hybridization.

### PRIOR ART DOCUMENTS

### PATENT DOCUMENTS

Patent Document 1: WO 2005/090997 A
Patent Document 2: JP 2007-212446 A
Patent Document 3: JP 2003-248008 A
Patent Document 4: JP 2003-339375 A

### SUMMARY OF THE INVENTION

### PROBLEMS TO BE SOLVED BY THE INVENTION

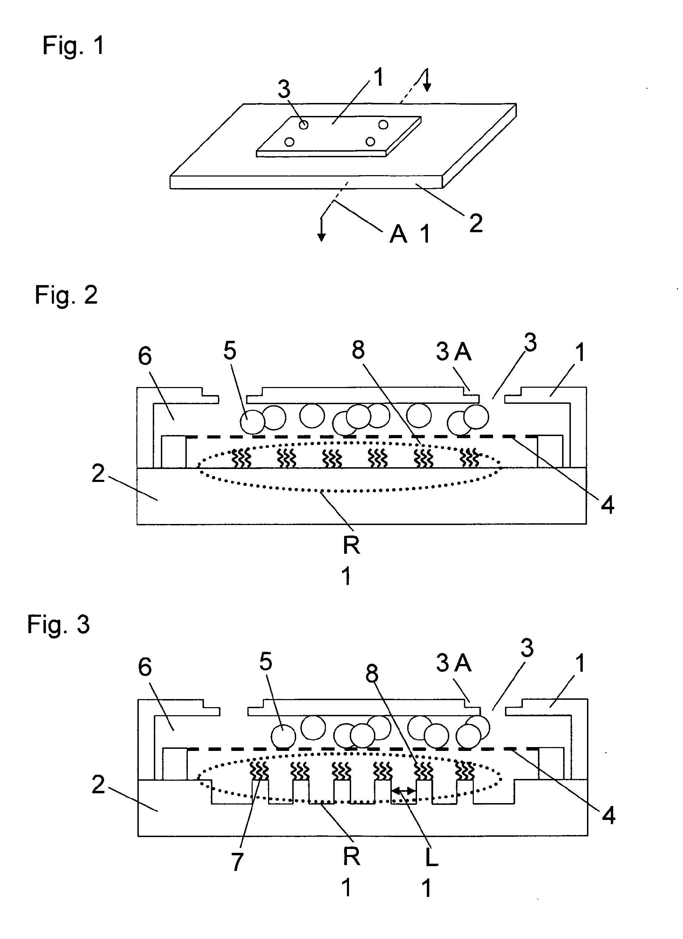

With the analysis chip disclosed in Patent Document 1, due to the structure provided to the carrier or the vessel in order to prevent the particles from contacting the portions on which the selective binding substances are immobilized, the size of the particles which may be used is limited. Therefore, it is not easy to use particles having a large size in order to obtain a comparatively large stirring force. Further, in case of the analysis chip shown in Fig. 2, since a high precision is required for the positioning in adhering the carrier and the vessel to produce the analysis chip, this production process may be very difficult.

With the analysis chip disclosed in the Patent Document 2, since the chip has a structure wherein wider recesses are provided between the subblock regions in addition to the recesses in the subblock regions, which both recesses are the regions in which the particles or the microrods are moved, the number of the protrusions which may be formed on the carrier is limited. Further, since the particles or the microrods are moved preferentially in the recesses between the subblock regions rather than in the recesses in the subblock regions, the stirring may be non-uniform.

With the analysis chip disclosed in Patent Document 3 and Patent Document 4, a plurality of magnetic beads may be coagulated by the magnetism to form a large block and may damage the selective binding substances immobilized on the surface of the substrate (carrier) or the efficiency of stirring may be decreased.

### MEANS FOR SOLVING THE PROBLEMS

To solve the above-described problems, the present invention has the following constitution:
[1] An analysis chip comprising:
   a carrier having a surface on which a selective binding substance(s) is(are) immobilized;
   a vessel holding a solution containing a test substance(s) which react(s) with the selective binding substance(s); and
   particles for stirring the solution, the particles being sealed within a space formed between the carrier and the vessel;
   wherein a separator which allows passage therethrough of the solution containing the test substance(s) but does not allow passage therethrough of the particles is arranged in the space so as to separate the surface of the carrier on which the selective binding substance(s) is(are) immobilized and the particles.
[2] The analysis chip according to [1], wherein the separator is a structure in the form of a sheet or plate having openings.
[3] The analysis chip according to [1], wherein the separator is a lattice-like structure.
[4] The analysis chip according to [1], wherein the separator is a mesh.
[5] A method of analyzing a test substance(s), the method comprising the steps of:
   contacting the test substance(s) with the analysis chip according to any one of [1] to [4];
   moving the particles to selectively bind the test substance(s) with the selective binding substance(s) on the carrier; and
   measuring amount of the test substance(s) bound to the carrier through the selective binding substance(s).
[6] A method of stirring a solution, the method comprising contacting the solution containing a test substance(s) which react(s) with a selective binding substance(s), with the selective binding substance(s) immobilized on a carrier surface; and stirring the solution with particles; wherein the particles are separated from the carrier surface on which the selective binding substance(s) is(are) immobilized, by a separator which allows passage therethrough of the solution containing the test substance(s) but does not allow passage therethrough of the particles.
[7] The method of stirring a solution, according to [6], wherein the separator is a structure in the form of a sheet or plate having openings.
[8] The method of stirring a solution, according to [6], wherein the separator is a lattice-like structure.
[9] The method of stirring a solution, according to [6], wherein the separator is a mesh.

### EFFECTS OF THE INVENTION

By the present invention, the damage of the selective binding substances due to the contact of the particles with the surface on which the selective binding substances are immobilized can be prevented during the reaction between the test substances and the immobilized selective binding substances while stirring the test substance solution with the particles.

Further, particles for stirring having a larger size than that of the conventionally used particles can be used irrespective of the shape of the carrier. As a result, the efficiency of stirring of the test substance solution can be promoted and the efficiency of the reaction between the test substances and the selective binding substances can be promoted, so that a high sensitive analysis chip by which the detection can be attained even when the amount of the sample is small can be provided.

Further, by the present invention, since entire surface on which the selective binding substances are immobilized in the analysis chip can be uniformly stirred without variation easily, the dispersion among the measurement data obtained from a plurality of immobilizing surfaces (spots) can be decreased.

Still further, with the analysis chip of the present invention, since the above-described object can be attained with a relatively simple structure, the analysis chip can be produced without a special consideration in the production process for attaining the above-described object.

### BRIEF DESCRIPTION OF THE DRAWINGS

Fig. 1 is a perspective view schematically showing an embodiment of the analysis chip of the present invention.
Fig. 2 is a cross-sectional view taken along the arrows A1 in Fig. 1 showing an embodiment of the analysis chip of the present invention.
Fig. 3 is a cross-sectional view taken along the arrows A1 in Fig. 1 showing an embodiment of the analysis chip of the present invention.
Fig. 4 is a perspective view schematically showing an embodiment of the analysis chip of the present invention.
Fig. 5 is a cross-sectional view taken along the arrows A2 in Fig. 4 showing an embodiment of the analysis chip of the present invention.
Fig. 6 is a cross-sectional view taken along the arrows A2 in Fig. 4 showing an embodiment of the analysis chip of the present invention.
Fig. 7 is a cross-sectional view taken along the arrows A2 in Fig. 4 showing an embodiment of the analysis chip of the present invention.
Fig. 8 is a cross-sectional view taken along the arrows A2 in Fig. 4 showing an embodiment of the analysis chip of the present invention.
Fig. 9 is a cross-sectional view taken along the arrows A2 in Fig. 4 showing an embodiment of the analysis chip of the present invention.
Fig. 10 is a perspective view schematically showing an embodiment of the analysis chip of the present invention.
Fig. 11 is a cross-sectional view taken along the arrows A3 in Fig. 10 showing an embodiment of the analysis chip of the present invention.
Fig. 12 is a cross-sectional view taken along the arrows A3 in Fig. 10 showing an embodiment of the analysis chip of the present invention.
Figs. 13(c) - (g) are perspective views of the separator constituting an embodiment of the analysis chip of the present invention.

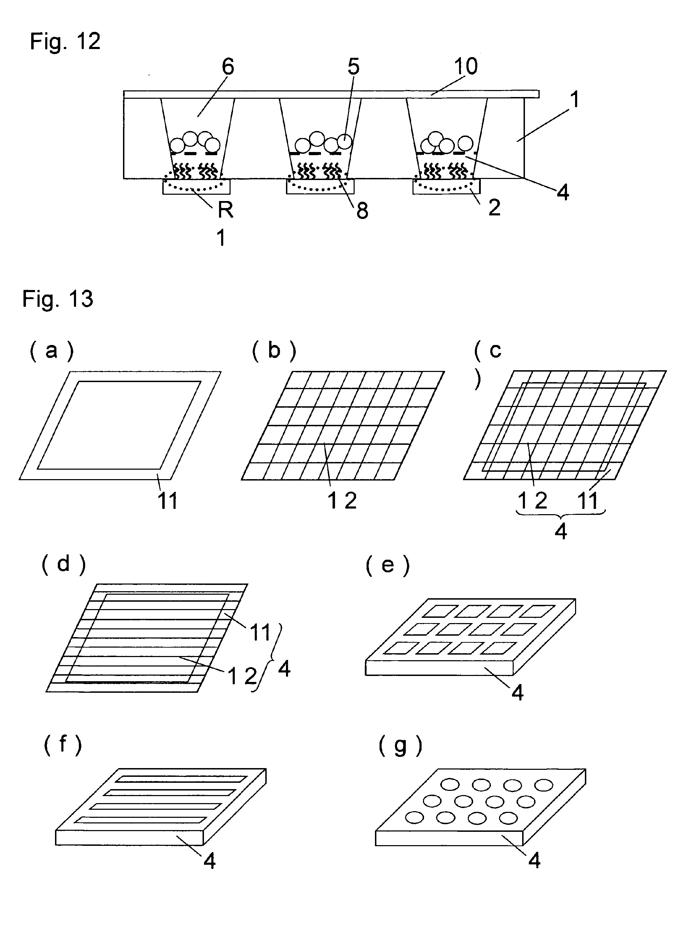

Figs. 13(a) and (b) show the outer frame and the member constituting the separator shown in Fig. 13(c) or (d).

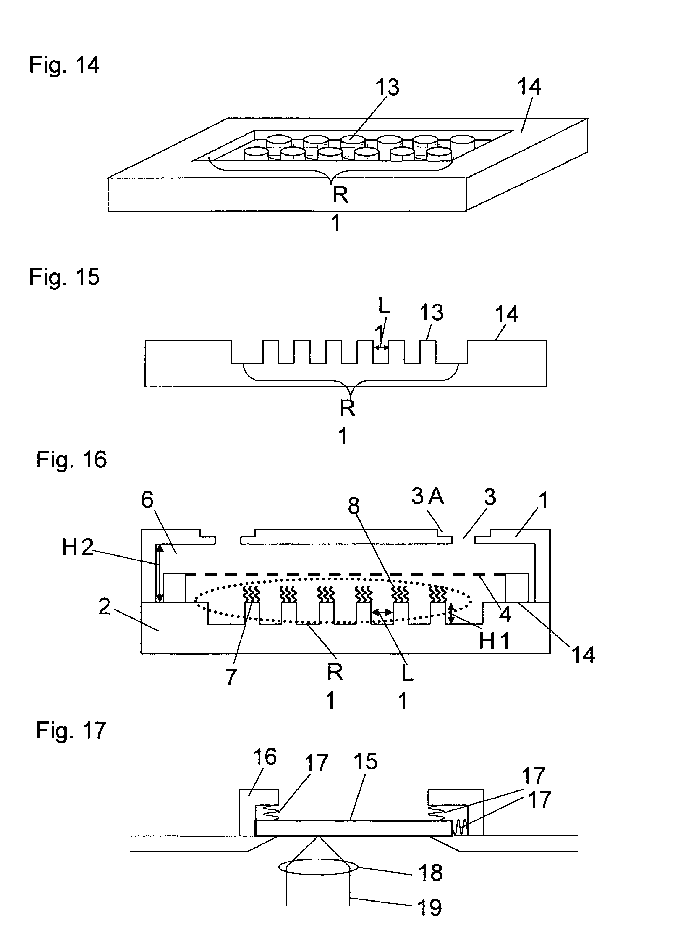

Fig. 14 is a perspective view schematically showing the carrier constituting an embodiment of the analysis chip of the present invention.
Fig. 15 is a longitudinal sectional view of the carrier illustrated in Fig. 14.
Fig. 16 is a cross-sectional view showing an embodiment of the analysis chip of the present invention.
Fig. 17 is a longitudinal sectional view conceptually showing an embodiment of the jig and the scanner reading the results of the reactions obtained using the analysis chip of the present invention.

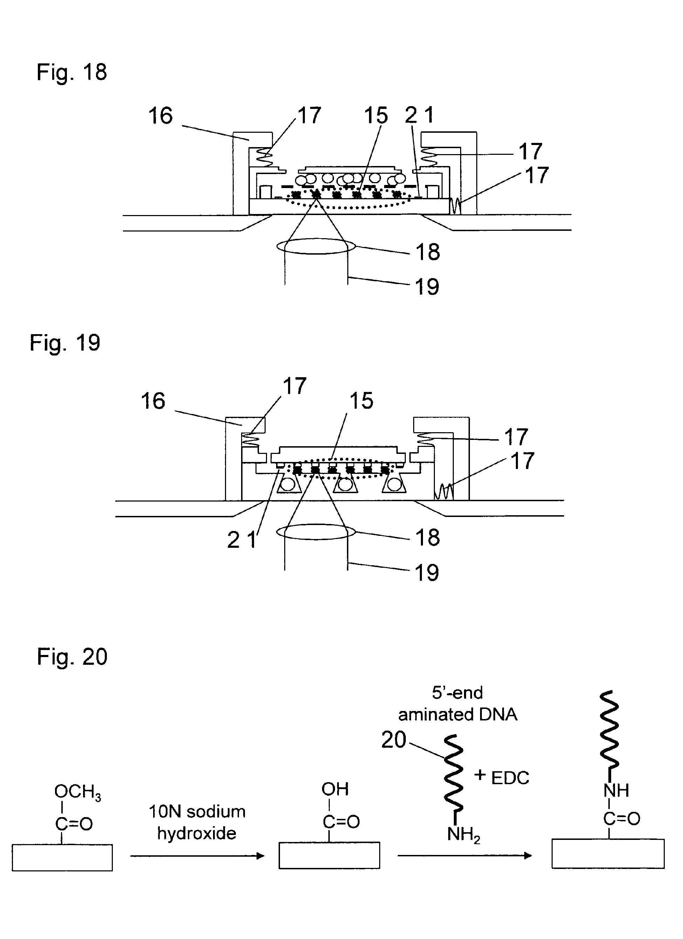

Fig. 18 is a longitudinal sectional view conceptually showing an embodiment of the jig and the scanner reading the results of the reactions obtained using the analysis chip of the present invention.
Fig. 19 is a longitudinal sectional view conceptually showing an embodiment of the jig and the scanner reading the results of the reactions obtained using the analysis chip of the present invention.
Fig. 20 is a schematic view showing the immobilization steps of the probe DNAs in the Examples of the present invention and in Comparative Examples.

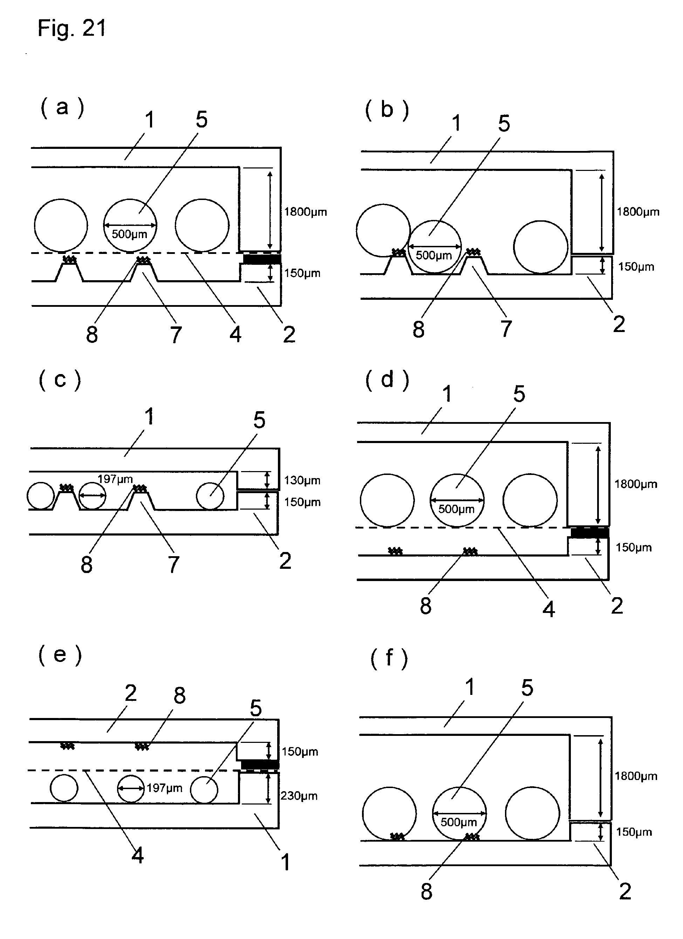

Figs. 21(a) - (f) are partial cross-sectional views of the analysis chips used in the Examples of the present invention and in Comparative Examples.

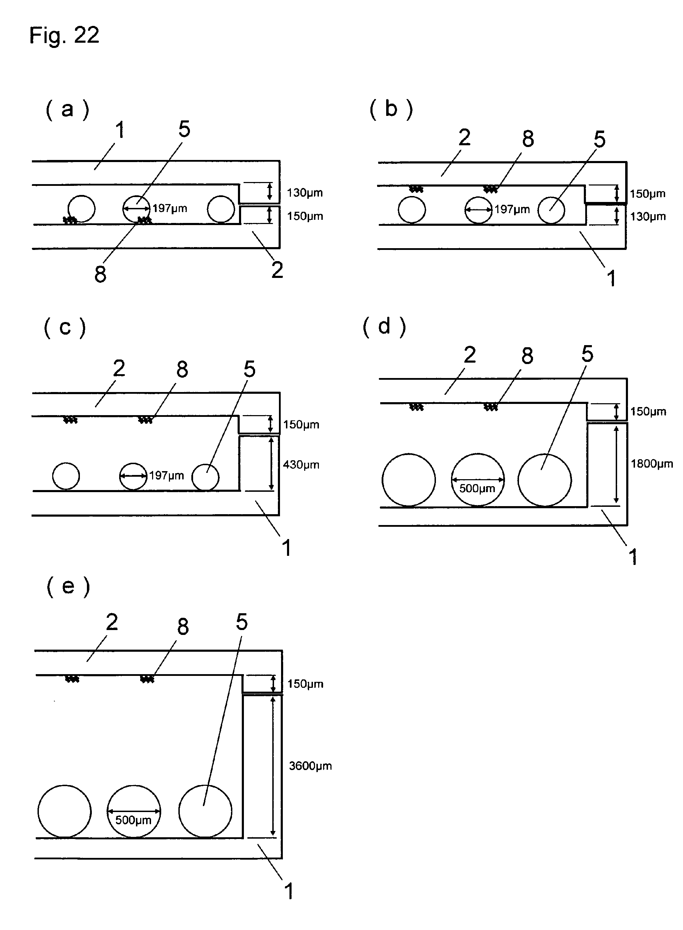

Figs. 22(a) - (e) are partial cross-sectional views of the analysis chips used in the Examples of the present invention and in Comparative Examples.

### MODE FOR CARRYING OUT THE INVENTION

The analysis chip of the present invention comprises a carrier having a surface on which a selective binding substance(s) is(are) immobilized; a vessel holding a solution containing a test substance(s) which react(s) with the selective binding substance(s); and particles for stirring the solution, the particles being sealed within a space formed between the carrier and the vessel; and has a separator arranged such that it separates the surface of the carrier on which the selective binding substance(s) is(are) immobilized and the particles. The separator arranged in the space allows passage therethrough of the solution containing the test substance(s) but does not allow passage therethrough of the particles for stirring.

Here, the carrier is a base material having a surface on which the selective binding substance(s) is(are) immobilized. The vessel is a base material covering the surface of the carrier on which the selective binding substance(s) is(are) immobilized, and is used in such a manner that it is adhered with the carrier so as to form a space holding the test substance solution.

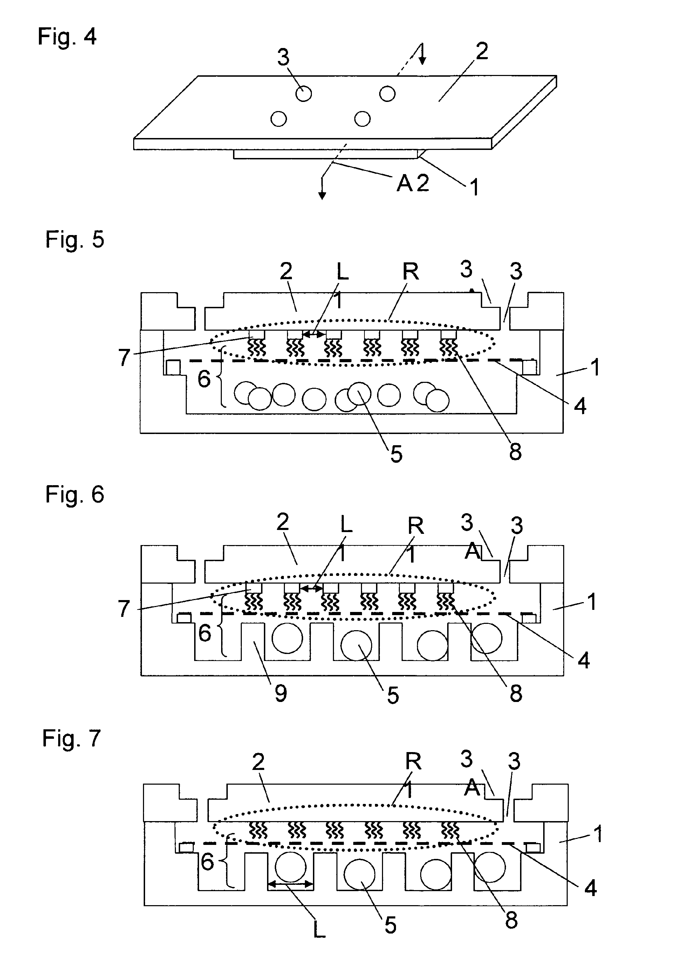

The analysis chip of the present invention includes, when it is used in the direction perpendicular to the direction of gravity (horizontal direction), those which can be used such that the surface of the carrier on which the selective binding substance(s) is(are) immobilized faces either upward (direction opposite to gravity) or downward (direction of gravity), and such that the surface faces the direction parallel to the direction of gravity (vertical direction). For example, all of Figs. 1 to 12 mentioned below show the cases where the chip is used in the direction (horizontal direction) perpendicular to the direction of gravity. In the analysis chips illustrated in Figs. 1 to 3 and Figs. 10 to 12, the surface on which the selective binding substances are immobilized faces upward. In this case, the vessel covers the carrier from the upper side. In the analysis chips illustrated in Figs. 4 to 9, the surface on which the selective binding substances are immobilized faces downward. In this case, the vessel covers the carrier from the lower side.

The analysis chip of the present invention is a chip used for detecting the existence of a test substance(s), for quantifying the test substance(s) or for measuring the properties or the like of the test substance(s) by applying a solution containing the test substance(s) to the chip. Examples thereof include biochips for measuring the amounts of the test substance(s) or detecting the existence of a test substance(s) based on the reactions between the selective binding substance(s) immobilized on the carrier surface and the test substance(s). Examples of the biochips include DNA chips wherein nucleic acids are immobilized on the carrier surface; protein chips wherein proteins represented by antibodies are immobilized on the carrier surface; sugar chain chips wherein sugar chains are immobilized on the carrier surface; and cell chips wherein cells are immobilized on the carrier surface.

First, specific modes of the analysis chip of the present invention are illustrated referring to the drawings.

In the embodiment of the analysis chip shown in Fig. 1, a vessel 1 covers a carrier 2 from upper portion thereof. Figs. 2 and Fig. 3 respectively show different modes of the analysis chip shown in Fig. 1, and are cross-sectional views taken along the arrows A1 in Fig. 1.

In the embodiments of the analysis chip shown in Fig. 2 and Fig. 3, the carrier 2 and the vessel 1 together form a space 6 including an immobilization region R1 on which selective binding substances are immobilized. The space 6 is a closed space which does not communicate with outside except that it may communicate via through holes 3 that may be formed as required. The immobilization region R1 in Fig. 2 is flat and R1 in Fig. 3 has an irregular structure.

The immobilization region R1 in the analysis chip shown in Fig. 2 and Fig. 3 is covered with a separator 4. Particles 5 for stirring are sealed within a region in the space, which region is separated from the immobilization surface of the selective binding substances by the separator 4, and move in the space above the separator 4.

In the embodiments of the analysis chip shown in Fig. 2 and Fig. 3, the particles 5 cannot pass through the separator 4, but a solution containing a test substance(s) can pass therethrough. Therefore, by moving the particles on the separator arranged in the space by subjecting the analysis chip to vibration, rotation or the like as described below after applying the solution containing the test substance(s) into the space, sufficient stirring of the solution is attained without contact of the moving particles 5 with the carrier surface on which the selective binding substances are immobilized. The flow of the solution caused thereby occurs through the separator and the solution in the entire space can be uniformly stirred. With the conventional analysis chips, since the carrier or the vessel has a structural device for preventing the particles from contacting the portions immobilizing the selective binding substances, the size of the particles which can be used is limited. In the present invention, by providing such a separator, the size of the particles is not basically limited.

In the embodiment of the analysis chip shown in Fig. 4, the vessel 1 covers the carrier 2 from the lower side. Fig. 5 to Fig. 9 respectively show different modes of the analysis chip shown in Fig. 4, and are cross-sectional views taken along the arrows A2 in Fig. 4.

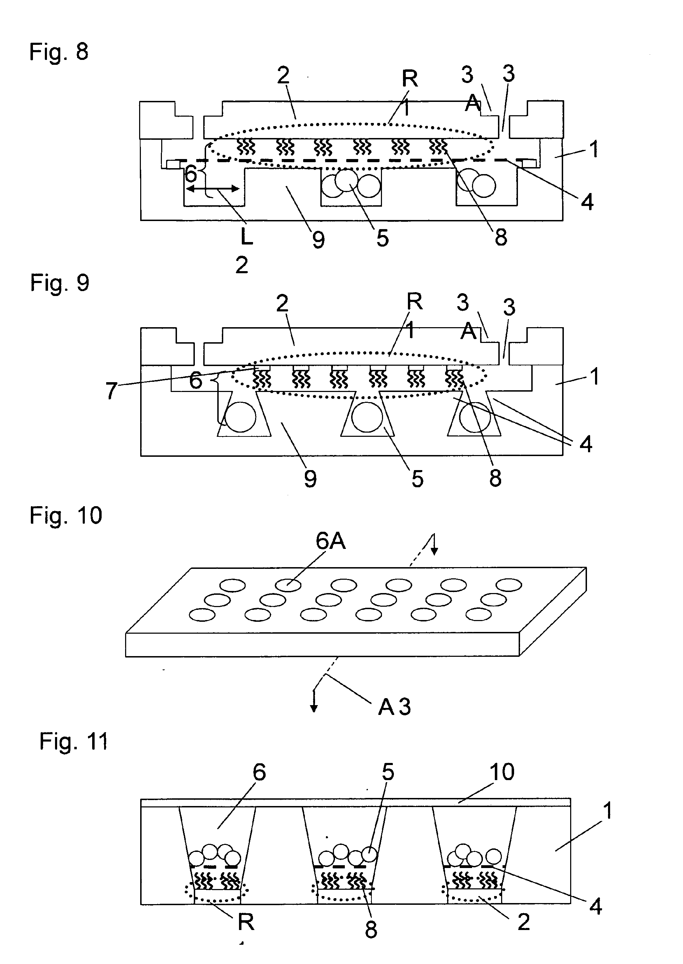

In the embodiments of the analysis chip shown in Fig. 5 to Fig. 9, the carrier 2 and the vessel 1 together form a space 6 including an immobilization region R1 on which selective binding substances are immobilized. The space 6 is a closed space which does not communicate with outside except that it may communicate via through holes 3 that may be formed as required. The surface of the carrier 2 shown in Fig. 5, Fig. 6 and Fig. 9 has an irregular structure, and the surface of the carrier 2 shown in Fig. 7 and Fig. 8 is flat. The surface of the vessel 1 shown in Fig. 5 is flat, and the surface of the vessel 1 shown in Fig. 6, Fig. 7, Fig. 8 and Fig. 9 has an irregular structure. In the irregular structure of vessel 1 shown in Fig. 8, the width of the protrusions is wider than that of the vessel 1 in Fig. 7. In the irregular structure of the vessel 1 shown in Fig. 9, the protrusions have a reverse tapered structure (reverse truncated cone structure).

The surface of the vessel 1 shown in Fig. 5, Fig. 6, Fig. 7 and Fig. 8 are covered with the separator 4. The surface of the vessel 1 shown in Fig. 9 is so designed that the size of the openings of the recesses is made smaller than the size of the particles 5 so that the particles 5 are retained in the spaces within the recesses due to the reverse tapered structure thereof. Thus, the reverse tapered structure itself plays the role as the separator 4.

The immobilization region R1 in the analysis chip shown in Fig. 5 to Fig. 9 is covered with the separator 4. Particles 5 are sealed within a region in the space, which region is separated from the immobilization surface of the selective binding substances by the separator 4, and move in the space below the separator 4.

In the embodiments of the analysis chip shown in Fig. 5 to Fig. 9, the particles 5 cannot pass through the separator 4, but a solution containing a test substance(s) can pass therethrough. Therefore, by moving the particles on the separator arranged in the space by subjecting the analysis chip to vibration, rotation or the like as described below after applying the solution containing the test substance(s) into the space, sufficient stirring of the solution is attained without contact of the moving particles 5 by jumping with the carrier surface on which the selective binding substances are immobilized. The flow of the solution caused thereby occurs through the separator and the solution in the entire space can be uniformly stirred.

In the conventional analysis chips, a high precision is required for the positioning of the irregular structure of the vessel in order to prevent the particles from contacting the portions on which the selective binding substances are immobilized. In contrast, in the analysis chip shown in Fig. 6 to Fig. 9, the strict positioning between the vessel and the carrier is not necessary because of using the separator of the present invention, so that it can be produced easily.

In the embodiments of the analysis chip shown in Fig. 10 to Fig. 12, the vessel 1 having well structures 6A is bound to the side surface of the carrier 2, or the bottom portion of the vessel 1 having the well structures 6A is bound to the surface of the carrier 2. A plurality of spaces 6 including the immobilization regions R1 on which the selective binding substances are immobilized are formed by the carrier 2 and the inner walls of the well structures of the vessel 1. The surface of the carrier 2 shown in Fig. 10 is flat.

The immobilization region R1 in the analysis chip shown in Fig. 10 to Fig. 12 is covered with a separator 4. Particles 5 are sealed within a region in the space, which region is separated from the immobilization surface of the selective binding substances by the separator 4, and move in the space above the separator 4. The upper portions of the spaces 6 may be open (not shown). In this case, after applying the solution, to prevent the flowing out of the particles 5 and the reaction solution, the spaces may be sealed with a sealing member 10 which is an adhesive tape or the like.

In the embodiments of the analysis chip shown in Fig. 10 to Fig. 12, the particles 5 cannot pass through the separator 4, but a solution containing a test substance(s) can pass therethrough. Therefore, by subjecting the analysis chip to vibration, rotation or the like after applying the solution containing the test substance(s) into the spaces, the flow of the solution caused thereby occurs through the separator and the solution in the entire spaces can be uniformly stirred.

As the separator of the analysis chip of the present invention, one through which the solution containing the test substance(s) can pass but the particles for stirring cannot pass may be employed. For example, a structure in the form of a sheet or plate having openings with a size such that the stirrer cannot pass through may be employed. In this case, the shape of the openings is not restricted, and may be rectangular such as square or oblong, circular, oval or the like.

For example, as the separator of the analysis chip of the present invention, a lattice-like structure having rectangular openings, or a structure in the form of a punching sheet as shown in Figs. 13(c) to (g) may be used. In the case of the lattice-like structure, the shape of the lattice windows may be various shapes, and those of which lattice windows are rectangular such as square or oblong, which are readily available, may be used. The separator 4 shown in Fig. 13(c) is a lattice-like mesh which is a lattice-like structure having rectangular openings, and the lattice-like member (mesh) is fixed to a separator outer frame 11 shown in Fig. 13(a) as a separator member 12. Fig. 13(d) shows a separator 4 constituted by a stripe-like member or a plurality of string-like member as a separator member 12 fixed to the separator outer frame 11 shown in Fig. 13(a). Further, a lattice-like structure or a structure in the form of a punching sheet having rectangular circular openings shown in Figs. (e) to (g) may be used.

The material of the separator is not restricted, and metals (e.g., gold, platinum and stainless steel), plastics (e.g., nylons, polystyrene, polyesters and polypropylene), carbon and the like may be employed. In cases where the material of the separator is a plastic, either of the plastic which is the same as the material of the carrier or the vessel, or a plastic different therefrom may be employed. As the separator outer frame 11 shown in Fig. 13(a), the material same as that of the separator member 12 (lattice-like member (mesh) or stripe-like member or string-like member) may be employed.

Among these, as the separator, the lattice-like mesh shown in Fig. 13(c) which is relatively easily available may preferably be used. Especially, as the separator member 12, a separator using a plastic mesh may preferably be used. As the material of the plastic mesh, plastics such as nylons, polystyrene, polyesters, polypropylene and fluorine fibers may be employed. Among these, polyesters and polypropylene are especially preferred. The wire diameter and the size of the openings of the mesh may be optimally selected depending on the size of the particles used, taking the ease of flowing of the solution and ease of moving of the particles into consideration.

The preferred sizes in cases where a lattice-like separator and spherical particles are used are now explained.

The size of the particles can be selected depending on the volume of the solution to be stirred containing the test substance(s). In the case of a DNA chip, taking the size of the vessel used in the preparation of the solution containing the test substance(s) and the volume of liquid which is easy to handle into consideration, the volume of the solution is usually about 1 mL at maximum. For example, in the case of the DNA chip shown in Fig. 16 described below, the selective binding substances are immobilized in an area of about 1 cm square. To bring a test substance solution of 1 mL into contact, the depth H2 of the recesses in the vessel holding the solution may be 1.8 mm. To attain a sufficient stirring, it is thought, in general, that the particles are preferably as large as possible. However, taking the size of the vessel holding the solution into consideration, in the embodiment of the above-described DNA chip, in cases where the particles are spherical, the upper limit of the size of the particles is preferably not more than 1.4 mm in terms of the average particle size. If the particles are larger than this, the friction with the separator and the vessel may be large and the particles may not move smoothly. In cases where the average particle size of the particles is less than 0.1 mm, if a separator having a mesh size which does not allow passage of the particles is used, the passing of the solution may also be hindered. Therefore, in case of using spherical particles, it is preferred to use particles having an average particle size within the range from 0.5 to 1.1 mm depending on the shape of the separator.

In cases where a plastic mesh is used as the lattice-like separator (separator member), preferred combinations of the size of the openings and the average particle size of the spherical particles are as follows: When the openings of the mesh is 0.05 mm to 0.09 mm, particles having an average particle size of 0.15 mm to 0.25 mm are preferably used; when the openings of the mesh is 0.1 mm to 0.25 mm, particles having an average particle size of 0.3 mm to 0.5 mm are preferably used; when the openings of the mesh is 0.25 mm to 0.35 mm, particles having an average particle size of 0.5 mm to 0.7 mm are preferably used; when the openings of the mesh is 0.35 mm to 0.45 mm, particles having an average particle size of 0.7 mm to 0.9 mm are preferably used; when the openings of the mesh is 0.45 mm to 0.55 mm, particles having an average particle size of 0.9 mm to 1.1 mm are preferably used. By properly using the mesh and the particles as described above, the particles can be prevented from being trapped within the openings of the mesh.

The wire diameter of the mesh is preferably as thin as possible in order not to hinder the flow of the solution as much as possible, and a mesh having a wire diameter of 0.06 mm to 0.16 mm is preferably used.

In cases where a lattice-like separator (separator member) is used, when the lattice shape is oblong, the rate of opening is larger and the flow of the solution is less hindered than the cases where the lattice shape is square. In cases where the lattice is oblong, it is preferred to select the optimal length of short side of the lattice window so that the particles are not trapped within the lattice windows in the separator. In case of spherical particles, when the average particle size of the particles is 0.15 mm to 0.25 mm, a lattice-like separator having a length of short side of 0.05 mm to 0.09 mm is preferably used; when the average particle size is 0.3 mm to 0.5 mm, the length of short side is preferably 0.1 mm to 0.25 mm; when the average particle size is 0.5 mm to 0.7 mm, the length of short side is preferably 0.25 mm to 0.35 mm; when the average particle size is 0.7 mm to 0.9 mm, the length of short side is preferably 0.35 mm to 0.45 mm; when the average particle size is 0.9 mm to 1.1 mm, the length of short side is preferably 0.45 mm to 0.55 mm. When the particles have an average particle size of 0.1 mm, the length of short side is too small so that the rate of opening is small, and so the flow of the solution is hindered. In the above-described cases, the length of the longer side of the oblong lattice is not specifically restricted.

To prevent corrosion of the separator it self and adsorption of the test substance(s) thereto, a surface modification such as physical coating or chemical modification may be applied to the separator.

The separator in the analysis chip of the present invention may be either removably adhered to the carrier or unremovably adhered to the carrier. Usually, after the hybridization reaction with the test substance(s), the intensity of the fluorescence emitted from the fluorescent substance bound to the selective binding substance(s) or the test substance(s) is measured with a scanner apparatus. In cases where the separator is removably adhered to the carrier, the analysis chip is disassembled, and after removing the separator and removing the vessel and the particles, the carrier alone is set in the scanner apparatus, and the signal intensity such as fluorescence can be measured.

On the other hand, it is possible to subject the analysis chip in which the separator is unremovably adhered to the carrier to the measurement with the scanner apparatus without disassembling the analysis chip. In this case, however, if self-fluorescence is emitted from the separator, this emission acts as a noise and the detection accuracy may be decreased. Therefore, it is important to use a separator which emits no or reduced self-fluorescence upon light irradiation. To decrease the self-fluorescence from the separator it self, it is preferred to constitute the separator with a polymer comprising the structural unit represented by the General Formula (1) or General Formula (2) described below; or with a black polymer incorporating a substance such as carbon black, carbon nanotube or fullerene, which substance does not emit light by light irradiation and/or which substance absorbs the light emitted from the polymer; or to coat such a polymer on the surface of the separator to attain a surface which does not emit light upon light irradiation. By using such a separator, for example, a black separator, which does not emit light upon light irradiation, the self-fluorescence from the separator during the detection can be decreased, so that the results having a high S/N ratio can be obtained.

Here, "the separator does not emit light upon light irradiation" means that the spectral reflectance of the separator does not have a specific spectral pattern (such as a specific peak) in the range of visible light (wavelength is 400 nm to 800 nm) and is uniformly a low value, and the spectral transmission of the separator also does not have a specific spectral pattern and is uniformly a low value.

In the analysis chip of the present invention, the separator preferably has a spectral reflectance of not higher than 7% in the range of visible light (wavelength is 400 nm to 800 nm) and preferably has a spectral transmission of not higher than 2% in this wavelength range. The "spectral reflectance" herein means the spectral reflectance wherein the regular reflection light from the carrier is taken by an illumination-light receiving optical system in accordance with Condition C of JIS Z 8722.

Preferred examples of the substance to be incorporated in the polymer which may be used in order to prevent the light emission from the separator upon light irradiation thereon include black substances such as carbon black, carbon nanotube, fullerene, graphite, titanium black and aniline black; oxides of Ru, Mn, Ni, Cr, Fe, Co and Cu; and carbides of Si, Ti, Ta, Zr and Cr. Among these black substances, carbon black, carbon nanotube, fullerene, graphite and titanium black may preferably be incorporated, and carbon black may especially preferably be employed. These black substances may be used individually or two or more of them may be incorporated in combination.

Preferred modes of the particles used in the analysis chip of the present invention are now described.

The size of the particles may be selected, as described above, such that the effect of stirring can be obtained depending on the volume of the solution containing the test substances to be stirred and on the shape of the separator. Especially, it is preferred to use particles having an average particle size of 0.5 to 1.1 mm in harmony with the shape of the separator.

In the analysis chip of the present invention, particles of any shape may be used as long as the solution can be stirred. Particles having any shape can be used as long as the test substance solution can be stirred by moving the particles. More specifically, the particles may have an optional shape such as sphere, polygon or cylinder. Among these, spherical particles, that is, beads are especially preferred. If the particles are spherical, the particles can be smoothly moved in the reaction solution without dwelling, so that stirring of the test substance solution can be well attained.

In the analysis chip of the present invention, the material constituting the particles is not restricted, and metals, glass, ceramics, polymers (such as polystyrene, polypropylene and nylons) may be employed. Among these materials, the materials having a specific gravity larger than water, for example, glass, quartz and zirconia ceramics, are preferred because the particles made of these materials can easily move in the solution by acceleration by gravity, vibration-permeation, rotation or the like, and the particle component does not elute into the test substance solution. It is also possible to use magnetic beads as the particles. Among these particles, the particles made of zirconia ceramics may most preferably be employed because they can move easily by applying an acceleration by gravity, vibration-shaking or rotation due to the high specific gravity. Further, glass, quartz and zirconia ceramics are preferred because a component of the particles is hardly eluted into the test substance solution.

Particles made of zirconia ceramics (yttria-stabilized zirconia) are especially preferred since they have a density of 6 g/cm³, which is larger than that of quartz glass whose density is 2.2 g/cm³, larger stirring effect can be obtained, and the beads are not raised upwards and moved by the movement of the solution during sealing with the vessel, so that the setting can be attained more easily.

With the analysis chip of the present invention, the solution is stirred by moving the particles. Preferably, the particles are moved by moving the particles utilizing the gravity or magnetic force (in case of magnetic beads), or by applying acceleration to the analysis chip by vibration-shaking or rotation, or combination thereof. Among these, the method wherein the particles are moved by rotating the analysis chip along the plane perpendicular to the direction of gravity, or by shaking (quickly moving) the analysis chip in the direction of up and down or back and forth is preferred because it can be easily performed and sufficient effect is obtained.

In the method wherein the analysis chip is rotated along the plane parallel or substantially parallel to the direction of gravity (the vertical or substantially vertical plane) to move the particles by gravity, the rotational speed of the analysis chip is preferably 0.1 rpm to 30 rpm. If the rotational speed is more than 30 rpm, the gravity in the opposite direction is applied before the particles are well moved in one direction. That is, the distance of reciprocation of the particles in the test substance solution is small, so that the effect of stirring may not be well exhibited. If the rotational speed is smaller than 0.1 rpm, the total time during which the particles move in the liquid is small, and the time during which the test substance solution is stirred is short, so that a sufficient effect may not be obtained. In view of these, more preferred range of the rotational speed is 0.5 rpm to 5 rpm.

A method wherein the analysis chip is revoluted along the plane perpendicular or substantially perpendicular to the direction of gravity (horizontal or substantially horizontal plane) to move the particles is also preferred because a commercially available shaker or the like can be utilized. The "substantially horizontal plane" means a plane having an inclination by which the particles do not gather in one side of the analysis chip when the carrier is rotated, and may preferably be a plane, for example, shifted from the horizontal plane by 0 to 3 degrees. "Revolution" means that the analysis chip revolutes about an optional revolution axis. In this case, it is preferred that the analysis chip revolute about an optional revolution axis such that it describes a circular orbit. Although the analysis chip itself may or may not rotate (spin), it is preferred that the analysis chip do not rotate because the apparatus is simple and a large acceleration or centrifugal force is applied, so that the efficiency of stirring is promoted.

The direction of revolution is not restricted, and the analysis chip may continuously revolute in one direction, or may revolute repeating a pattern switching the forward direction and reverse direction at a constant period such as per one or two revolutions, or at a random period. The rate of revolution when the analysis chip is revoluted is preferably from 100 rpm to 500 rpm. If the rate of revolution is less than 100 rpm, sufficient stirring may not be performed, and if it is more than 500 rpm, the vessel may be peeled off from the analysis chip by the centrifugal force. The rate of revolution is especially preferably from 200 rpm to 300 rpm. The rate of revolution may be constant throughout the stirring time, or may be changed periodically or randomly. The radius of the circular orbit described by the analysis chip during the revolution is preferably from 2 cm to 5 cm in terms of the radius of the circular orbit described by the central portion of the analysis chip. If the radius is less than 2 cm, sufficient stirring may not be performed, and if it is more than 5 cm, the vessel may be peeled off from the analysis chip by the centrifugal force. The circular orbit is not restricted to perfect circle, but may have more or less deformation, and may be, for example, ellipse.

A method wherein the analysis chip is swung describing the figure of 8 in the plane perpendicular or substantially perpendicular to the direction of gravity (horizontal or substantially horizontal plane) to move the particles is also preferred. In this case, it is preferred that the analysis chip describe figure of 8 left-right symmetrically about a figure of 8 swing axis. The direction of the figure of 8 swing is not restricted, and the analysis chip may continuously swing describing figure of 8 in one direction, or may swing describing figure of 8 repeating a pattern switching the forward direction and reverse direction at a constant period such as per one or two swings, or at a random period. The rate of swing when the analysis chip is swung is preferably from 100 rpm to 500 rpm. If it is less than 100 rpm, sufficient stirring may not be performed, and if it is more than 500 rpm, the vessel may be peeled off from the analysis chip by the centrifugal force. The rate of swing is especially preferably from 200 rpm to 300 rpm. The rate of figure of 8 swing may be constant throughout the stirring time, or may be changed periodically or randomly. In the figure of 8 swing, the radius of the figure of 8 orbit described by the analysis chip during the swing is preferably from 2 cm to 5 cm in terms of the radius of the circular orbit described by the central portion of the analysis chip. If the radius is less than 2 cm, sufficient stirring may not be performed, and if it is more than 5 cm, the vessel may be peeled off from the analysis chip by the centrifugal force.

A method wherein acceleration is applied by vibrating or shaking the analysis chip to move the particles in the solution is also a method which may preferably be employed. In this case, it is preferred to shake the analysis chip up-down symmetrically or left-right symmetrically about an optional axis. When the analysis chip is shaken up and down or back and forth, the rate of shaking is preferably from 100 times/min to 500 times/min. If the rate of shaking is less than 100 times/min, sufficient stirring may not be performed, and if it is more than 500 times/min, the vessel may be peeled off from the analysis chip by the centrifugal force. The rate of shaking is especially preferably from 200 times/min to 300 times/min. The rate of shaking may be constant throughout the stirring time, or may be changed periodically or randomly. In the shaking, the amplitude of shaking of the analysis chip is preferably 2 cm to 5 cm. If the amplitude is less than 2 cm, sufficient stirring may not be performed, and if it is more than 5 cm, the vessel may be peeled off from the analysis chip by the centrifugal force.

In cases where the fluorescence intensity is measured by a scanner apparatus after the hybridization reaction with the test substances without disassembling the analysis chip and removing the vessel or the separator, if self-fluorescence is emitted from the particles, this emission acts as a noise and the detection accuracy may be decreased. Therefore, it is important to use a separator which emits no or reduced self-fluorescence upon light irradiation. To decrease the self-fluorescence from the particles themselves, similar to the case of separator mentioned above, it is preferred to constitute the particles with a polymer comprising the structural unit represented by the General Formula (1) or General Formula (2) described below; or with a black polymer incorporating a substance such as carbon black, carbon nanotube or fullerene, which substance does not emit light by light irradiation and/or which substance absorbs the light emitted from the polymer; or to coat such a polymer on the surface of the particles to attain a surface which does not emit light upon light irradiation. By using such particles, the self-fluorescence from the particles can be reduced during the detection.

Preferred shape of the carrier on which the selective binding substance(s) is(are) immobilized is now described.

As the carrier on which the selective binding substance(s) is(are) immobilized used in the analysis chip of the present invention, those whose surface is flat or has an irregular structure can be used. In cases where a carrier whose surface has an irregular structure is used, it is preferred that the selective binding substance(s) be immobilized on the upper surface of the protrusions in the irregular structure. By employing such a structure, the detection of the non-specifically adsorbed test substance(s) can be reduced, so that the noise is small and good results having low S/N can be obtained. The concrete reason why the noise is made small is as follows: That is, when the carrier on which the selective binding substance(s) is(are) immobilized on the upper surface of the protrusions is scanned with an apparatus called a scanner, since the laser light is focused on the upper surface of the protrusions in the irregular structure in either cases where the detection is conducted from the surface of the carrier (the surface on which the selective binding substance(s) is(are) immobilized) or where the detection is conducted from the backside, the laser light is blurred at the recesses, so that the undesirable fluorescence (noise) from the test substance(s) non-specifically adsorbed to the recesses is hardly detected.

The heights of the protrusions in the irregular structure are preferably substantially identical. Here, "the heights are substantially identical" means that the variations in the intensities of the signal level are not problematic when the selective binding substance(s) is(are) immobilized on the surface of the protrusions whose heights are more or less different, the selective binding substance(s) is(are) reacted with the test substance(s) and the resultant is scanned with a scanner. More concretely, "the heights are substantially identical" means that the difference in height is less than 50 µm. More preferably, the difference in height is less than 30 µm, and still more preferably, the height is the same.

Further, the upper surface of the protrusions is preferably substantially flat. Here, "the upper surface of the protrusions is substantially flat" means that irregularities of not less than 20 µm do not exist.

Further, among the analysis chips whose carrier has an irregular structure, those analysis chips with which the detection is carried out from the side of the irregular surface on which the selective binding substance(s) is(are) immobilized preferably have a flat portion at the periphery thereof. Still further, it is preferred that the height of the upper surface of the protrusions in the irregular portion and the height of the flat portion be substantially the same. That is, the difference in the height of the flat portion and the height of the upper surface of the protrusions is preferably not more than 50 µm. More preferably, the different is not more than 30 µm, most preferably, the height of the flat portion and the height of the protrusions are the same.

An embodiment of a carrier having the irregular structure of the analysis chip of the present invention is illustrated in Fig. 14, Fig. 15 and Fig. 16. At the periphery of the irregular portion, a flat portion 14 is arranged, and the selective binding substances (e.g., nucleic acids) are immobilized on the upper surfaces 13 of the protrusions. With such a carrier, when the detection is carried out from the side of the irregular surface, the excitation light can be easily focused on the upper surfaces of the protrusions using this flat portion. More particularly, when the scanner focuses the excitation light on the surface of the carrier, the focusing is often preliminarily carried out such that a carrier 15 is abutted to a jig 16, and a laser light 19 is focused on the abutting surface of the jig 16, as shown in Fig. 17. By abutting the flat portion of the carrier used in the analysis chip of the present invention to the surface of the jig, the laser light from the scanner can easily be focused on the upper surface of the protrusions.

As shown in Fig. 18, in cases where the surface of the carrier on which the selective binding substance(s) is(are) immobilized is flat, and where the signals are read from the backside of the surface on which the selective binding substance(s) is(are) immobilized without disassembling the analysis chip and removing the vessel or the separator, in order to focus the laser light on the surface on which the selective binding substance(s) is(are) immobilized, it is preferred to provide, for example, a marker region on the carrier, which enables the surface to be detected by reflection or the like. In cases where the analysis chip illustrated in Fig. 9 is read by a scanner without removing the vessel, as shown in Fig. 19, it is preferred to provide a marker region on the carrier, which enables the surface to be detected by reflection or the like, which surface is substantially the same height as the surfaces on which the selective binding substance(s) is(are) immobilized, in order to focus the laser light on the surface, and to read the signals by a scanner from the side of the vessel.

In the analysis chip of the present invention, the "immobilization surface" on which the selective binding substance(s) is(are) immobilized means the portion on which the selective binding substance(s) (e.g., nucleic acid) is(are) immobilized from which data are necessary, and the portions on which a selective binding substance nothing more than a dummy is immobilized are excluded.

In the analysis chip of the present invention, either in the case where the surface shape of the regions on which the selective binding substance(s) is(are) immobilized are flat or irregular, it is preferred that the areas of the respective surfaces on which the selective binding substance(s) is(are) immobilized be substantially the same. Here, "the areas of the immobilization surfaces are substantially the same" means that the quotient obtained by dividing the largest area of the immobilization surface by the smallest area of the immobilization surface is not more than 1.2.

In the carrier having the irregular structure, the area of the upper surface of the protrusions on which the selective binding substance(s) is(are) immobilized is not restricted, and is preferably not larger than 1 mm², and not less than 10 µm², from the viewpoint of reducing the amount of the selective binding substance(s) and ease of handling.

In the carrier having the irregular structure, the height of the protrusions is preferably not less than 10 µm and not more than 500 µm, especially preferably not less than 50 µm and not more than 300 µm. If the height of the protrusions is smaller than this, the test substance(s) non-specifically adsorbed on portions other than the spots (protrusion surfaces) may be detected, and as a result, the S/N may be lowered. On the other hand, if the height of the protrusions is not less than 500 µm, the protrusions are likely to be snapped and broken, which is problematic.

The material constituting the carrier used in the analysis chip of the present invention is not restricted. Preferred materials of the carrier are glass and various polymers. Examples of the polymer include polystyrene, polymethyl methacrylate, polycarbonate and polyolefins. The term "polymer" herein means a macromolecular compound having a number average degree of polymerization of not less than 50. The preferred range of the number average degree of polymerization of the polymer is from 100 to 10,000. Especially preferably, the number average degree of polymerization of the polymer is not less than 200 and not more than 5000. The number average degree of polymerization can be easily measured by measuring the molecular weight of the polymer by a conventional method using GPC (gel permeation chromatography).

In cases where the material of the carrier is a glass, the immobilization of the selective binding substance can be attained by, for example, performing silane coupling treatment to generate functional groups on the surface, and the selective binding substance such as DNA can be immobilized using the functional groups as the toehold. For example, amino groups can be generated on the surface of the glass using an aminoalkylsilane, and in case of DNA, the DNA can be immobilized utilizing the positive charge of the amino groups and the negative charge of the DNA.

In the present invention, it is preferred that the carrier surface for immobilizing the selective binding substance be a solid containing a polymer having a structural unit represented by General Formula (1) below because the self-fluorescence of the carrier upon light irradiation can be reduced.

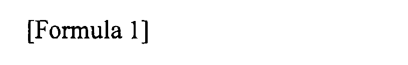

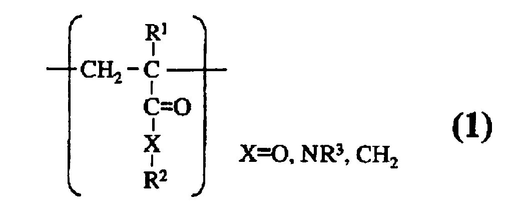

(wherein in General Formula (1), R¹, R² and R³ represents an alkyl group, aryl group or a hydrogen atom).

As the polymer containing the structural unit represented by General Formula (1), a homopolymer or a copolymer can be employed. In case of a copolymer, the copolymer preferably contains the structural unit represented by General Formula (1) in an amount of not less than 10% based on the total monomer units.

In General Formula (1), R¹ and R² represent, the same or different, an alkyl group, an aryl group or a hydrogen atom. The alkyl group may be straight or branched and preferably has a carbon number of 1 to 20. The aryl group preferably has a carbon number of 6 to 18, more preferably 6 to 12. Functional group X is optionally selected from O, NR₃ and CH₂. R³ is a functional group having the same definition as R¹ and R².

As the polymer constituting the carrier surface, a thermoplastic copolymer having an acid anhydride unit may also be employed. The thermoplastic copolymer preferably has an acid anhydride unit. The acid anhydride unit herein means the unit existing in the skeleton of the main chain or a side chain of the thermoplastic copolymer or at the terminal. Examples of the acid anhydride unit include (meth)acrylic anhydride unit, glutaric anhydride unit, maleic anhydride unit, itaconic anhydride unit, citraconic anhydride unit and aconitic anhydride unit. Among these, maleic anhydride unit and glutaric anhydride unit are preferred, and the glutaric anhydride unit represented by the General Formula (2) below is more preferred.

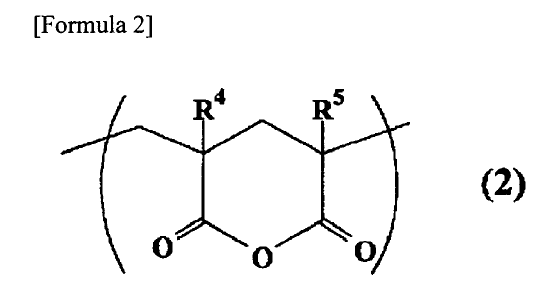

(wherein R⁴ and R⁵, the same or different, represent a hydrogen atom or a C₁-C₅ alkyl group).

Although the thermoplastic copolymer is not restricted as long as it contains the acid anhydride unit, the thermoplastic copolymer preferably has an unsaturated carboxylic acid unit represented by General Formula (3) below.

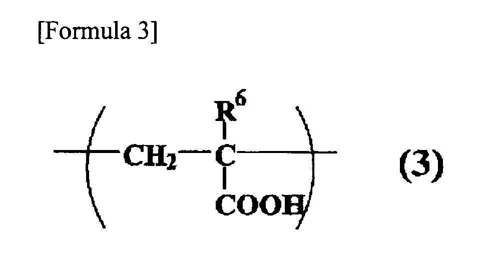

(wherein R⁶ represents a hydrogen atom or a C₁-C₅ alkyl group).

The "unsaturated carboxylic acid unit" herein means the unit obtained by copolymerizing an unsaturated carboxylic acid monomer. The unsaturated carboxylic acid monomer used here is not restricted, and any unsaturated carboxylic acid monomer which can be copolymerized with another vinyl compound can be employed. Examples of the preferred unsaturated carboxylic acid monomer include those represented by the General Formula (4) below,

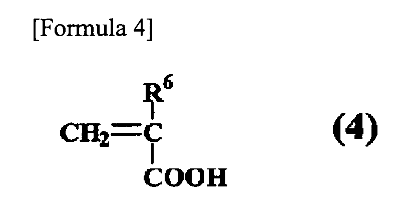

(wherein R⁶ represents a hydrogen atom or a C₁-C₅ alkyl group). maleic acid and hydrolysate of maleic anhydride. From the viewpoint of the excellent heat stability, acrylic acid and methacrylic acid are preferred, and methacrylic acid is more preferred. These units may be employed individually or two or more of them may be employed in combination.

Further, the thermoplastic copolymer preferably has an unsaturated carboxylic acid alkyl ester unit represented by the General Formula (5) below.

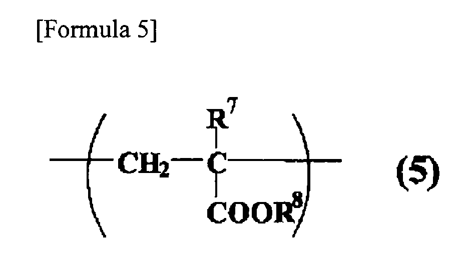

(wherein R⁷ represents a hydrogen atom or a C₁-C₅ alkyl group; R⁸ represents a C₁-C₆ aliphatic or alicyclic hydrocarbon group, or C₁-C₆ aliphatic or alicyclic hydrocarbon group substituted by halogen). The "unsaturated carboxylic acid alkyl ester unit" herein means a unit obtained by copolymerizing an unsaturated carboxylic acid alkyl ester monomer. Here, the unsaturated carboxylic acid alkyl ester monomer is not restricted, and preferred examples include those represented by the General Formula (6) below. (wherein R⁷ and R⁸ have the same definitions as described above).

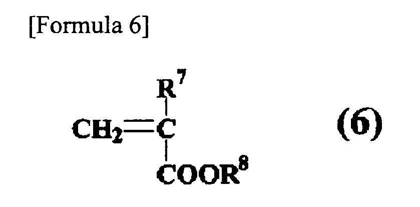

In the analysis chip of the present invention, the carrier surface for immobilizing the selective binding substance is preferably a polymer having functional groups. If the surface of the carrier made of the polymer has carboxylic groups or acid anhydride as functional groups, the selective binding substance having an amino group or hydroxyl group can be immobilized on the carrier surface by covalent bond. In cases where carboxylic groups exist on the carrier surface, to enhance the immobilization reaction of the selective binding substance, various condensing agents such as dicyclohexylcarbodiimide, N-ethyl-5-phenylisooxazolium-3'-sulfonate, 1-ethyl-3-(3-dimethylaminopropyl)carbodiimide (EDC) can be used. Using such a condensing agent, when the carboxylic group on the carrier surface and the amino group on the selective binding substance are reacted, the selective binding substance is immobilized on the carrier surface through an amide bond, and when the carboxylic group on the carrier surface and the hydroxyl group on the selective binding substance are reacted, the selective binding substance is immobilized on the carrier surface through an ester bond. In cases where a polymer in which an acid anhydride exists on the surface, the above-described condensing agent may be added, but even if the condensing agent is not added, covalent bond can be attained, for example, with an amino group on the selective binding substance.

It is preferred to immobilize the selective binding substance on the carrier whose surface is a polymer because non-specific adsorption of the test substance is reduced, and the selective binding substance can be firmly immobilized through a covalent bond at a high density. In cases where the carrier surface is a polymer, a carrier having a higher hybridization efficiency with the test substance can be obtained than in the cases where the carrier is made of glass presumably because the spacial freedom of the immobilized selective binding substance is larger.

Preferred examples of the polymer having functional groups include polyalkyl methacrylates (PAMA) such as polymethyl methacrylate (PMMA), polyethyl methacrylate (PEMA) and polypropyl methacrylate. Among these, especially preferred is polymethyl methacrylate. Further, polyvinyl acetate, polycyclohexyl methacrylate, polyphenyl methacrylate or the like can also be employed. Still further, copolymers containing the structural units of these polymers in combination, or the copolymers having a structure containing the structural units of these polymers together with the structural units of one or more other polymers can also be employed. Here, as the other polymer, polystyrene is exemplified. In cases where the polymer is a copolymer, the percentage of the monomer(s) containing a carbonyl group, such as an alkyl methacrylate, is preferably not less than 10 mol%.

To immobilize a selective binding substance on a carrier having on its surface the polymer containing at least one structural unit represented by General Formula (1), it is preferred to perform a pretreatment to generate carboxylic groups on the carrier surface. Examples of the method of generating carboxylic groups on the carrier surface include treatments with a liquid or solution of an alkali or acid; sonication in warm water; and exposure of the carrier to oxygen plasma, argon plasma or radiation. From the viewpoint of easily carrying out the method, the method wherein the carrier is immersed in a liquid or solution of an alkali or acid to generate carboxylic groups on the surface is preferred. For example, the carrier is immersed in an aqueous solution (preferred concentration is 1N to 20N) of sodium hydroxide or sulfuric acid, and incubated preferably at 30°C to 80°C for 1 hour to 100 hours.

Since the carrier whose surface on which the selective binding substance is immobilized has an irregular structure can be prepared by injection molding or hot embossing if the polymer containing the structural unit represented by General Formula (1) or General Formula (2) is used, a carrier having a precise irregular shape can be easily produced in a large scale. Especially, since injection molding enables large scale production, it can preferably be employed.

In the measurement of a test substance using the analysis chip of the present invention, usually, a fluorescently labeled test substance and the selective binding substance immobilized on the carrier are subjected to hybridization reaction, and thereafter, the fluorescence intensity is measured by a scanner apparatus. Here, the scanner diaphragms the laser light which is the excitation light with an objective lens, and condenses the laser light. However, if self-fluorescence is generated from the carrier surface, the emission serves as a noise and detection accuracy may be reduced. Therefore, it is important to use a carrier which emits no or reduced self-fluorescence upon light irradiation. To decrease the self-fluorescence from the carrier itself, similar to the case of separator mentioned above, it is preferred to constitute the carrier with a polymer comprising the structural unit represented by the General Formula (1) or General Formula (2); or with a polymer (e.g., the above-described black polymer) incorporating a substance which does not emit light by light irradiation or which substance absorbs the light emitted from the polymer; or to coat such a polymer on the surface of the carrier to attain a surface which does not emit light upon light irradiation. By using such a carrier which does not emit light upon light irradiation, the self-fluorescence from the carrier can be reduced during the detection, and a carrier with which a high S/N ratio is resulted can be attained. The definition that the carrier is black, and the method of making the carrier black are the same as in the separator described above.

As the shape of the vessel in the analysis chip of the present invention, one which enables to cover the immobilization surface of the carrier on which the selective binding substance(s) is(are) immobilized, and enables to be adhered such that it gives a space between the carrier and the vessel can be selected.

The analysis chip of the present invention comprises a space formed by the carrier and the vessel. The space may be one space, or may be a plurality of partitioned spaces. The plural partitioned spaces can be provided by a partitioning structure as in the analysis chip as shown in, for example, Fig. 11. In this embodiment, spaces 6 do not communicate each other. By providing a plurality of partitioned spaces as such, a plurality of types of test substance solution can be applied to one analysis chip, so that a plurality of types of test substance can be measured simultaneously with one chip.

The vessel in the analysis chip of the present invention may be provided with an irregular structure as, for example, the analysis chip shown in Fig. 6, Fig. 7 or Fig. 8. The vessel having the irregular structure can be prepared by simple cutting or by a usual injection molding. Further, the reverse tapered structure as in the analysis chip shown in Fig. 9 can be produced by a known method such as two-color molding.

The vessel in the analysis chip of the present invention may be removably adhered to the carrier or unremovably adhered to the carrier. In cases where the vessel is removably adhered to the carrier, after the hybridization reaction with the test substance(s), the analysis chip may be disassembled and the vessel may be removed, and the signal intensity can be measured by setting the carrier alone.

The material of the vessel constituting the analysis chip of the present invention is not restricted, and preferred examples of the materials to be used include glass and plastics as well as combinations thereof. From the viewpoint that structures such as through holes and liquid surface-halting chamber can be easily prepared, polymers such as polystyrene, polymethyl methacrylate and polycarbonate may preferably be employed.

To make it possible to observe the state of the solution when the test substance solution is applied, as the material of the vessel, a transparent material is preferred. In the analysis chips with which the signals are read by the scanner without removing the vessel from the analysis chip, the portion of the vessel through which the light passes should be constituted by a sufficiently transparent material. Further, if self-fluorescence is generated from the vessel, the self-fluorescence serves as a noise, which may lead to the decrease in the detection accuracy.

In cases where the detection is carried out without removing the vessel from the analysis chip and without passing the light through the vessel, to decrease the self-fluorescence from the vessel itself, similar to the case of separator or the carrier mentioned above, it is preferred to constitute the vessel with a polymer comprising the structural unit represented by the General Formula (1) or General Formula (2); or with a polymer (e.g., the above-described black polymer) incorporating a substance which does not emit light by light irradiation or which substance absorbs the light emitted from the polymer; or to coat such a polymer on the surface of the carrier to attain a surface which does not emit light upon light irradiation. By using such a vessel, e.g., a black vessel, which does not emit light upon light irradiation, the self-fluorescence from the vessel can be reduced during the detection. The definition that the vessel is black, and the method of making the vessel black are the same as in the separator or the carrier described above.

On the other hand, in cases where the detection is carried out by irradiating the light through the vessel, it is preferred to form the vessel with the polymer containing the structural unit represented by General Formula (1) or General Formula (2) or with non-fluorescent glass, which does not generate self-fluorescence and is transparent, or to form the vessel using, in combination, such a material only in the portion through which the light passes, and a material which does not generate self-fluorescence upon irradiation of light for forming other portions.

The method of preparing the vessel is not restricted, and the portions constituted by a polymer can be prepared by cutting or by injection molding. From the viewpoint that large scale production is possible, injection molding may preferably be employed.

In the present invention, "selective binding substance" means a substance which can selectively bind to the test substance directly or indirectly. Representative examples of the selective binding substance include nucleic acids, proteins, saccharides and other antigenic compounds.

As the selective binding substance, especially preferred are nucleic acids. The nucleic acid may be any of DNA, RNA and PNA. Since a single-stranded nucleic acid having a particular base sequence selectively hybridizes and binds with a single-stranded nucleic acid complementary to the base sequence or to a part thereof, it is a selective binding substance defined in the present invention. Examples of the protein include antibodies, antigen-binding fragments of antibodies such as Fab fragments and F(ab')2 fragments, and various antigens. Since an antibody or an antigen-binding fragment thereof selectively binds to the corresponding antigen, and an antigen selectively binds to the corresponding antibody, they are the "selective binding substance". As the saccharide, polysaccharides are preferred, and various antigen can be exemplified. Further, an antigenic substance other than proteins and saccharides may be immobilized as the selective binding substance.

The selective binding substance used in the present invention may be one commercially available or one obtained from a live cell or the like.

The selective binding substance used in the present invention is preferably a nucleic acid. Among the nucleic acids, one having a length of from 10 bases to 100 bases, called oligonucleic acid, is preferred because it can be easily synthesized artificially by a synthesizer, and immobilization on the carrier surface is easy because the end of the nucleic acid is easy to be modified with an amino group. In view of the fact that if the size of the nucleic acid is less than 20 bases, the stability of the hybridization is low, one having a size of 20 to 100 bases is preferred. To retain the stability of hybridization, the size is especially preferably within the range from 40 to 100 bases.

The method of analyzing a test substance(s) according to the present invention is a method comprising the steps of contacting the test substance(s) with the above-described analysis chip according to the present invention; moving the particles to selectively bind the test substance(s) with the selective binding substance(s) on the carrier; and measuring amount of the test substance(s) bound to the carrier through the selective binding substance(s).

In case of the analysis chip, for example, illustrated in Fig. 1 to Fig. 9, the solution containing the test substance(s) can be applied into the space via the through hole 3. By forming a plurality of through holes in the analysis chip, inflow of bubbles can be reduced when applying the solution containing the test substance(s), so that the operability is promoted.

In the analysis method of the present invention, the step of moving the particles to stir the solution containing the test substance(s) may preferably be attained by moving the particles utilizing the gravity or magnetic force (in case of magnetic beads), or by applying acceleration to the analysis chip by vibration-shaking or rotation, or combination thereof. Among these, the method wherein the particles are moved by rotating the analysis chip along the plane parallel or substantially parallel to the direction of gravity (the vertical or substantially vertical plane), thereby moving the particles by gravity is preferred because it can be easily performed and sufficient effect is obtained. In this case, the conditions of rotation such as the rotational speed are those described above. Further, as described above, the method wherein the analysis chip is swung describing the figure of 8 in the plane perpendicular or substantially perpendicular to the direction of gravity (horizontal or substantially horizontal plane) to move the particles is also preferred.

The method of stirring a solution according to the present invention is a method comprising contacting the solution containing a test substance(s) which react(s) with a selective binding substance(s) immobilized on a carrier surface; and stirring the solution with particles; wherein the particles are separated from the carrier surface on which the selective binding substance(s) is(are) immobilized, by a separator. The separator used here allows passage therethrough of the solution containing the test substance(s) but does not allow passage therethrough of the particles. The method of stirring a solution according to the present invention may be effectively used when the test substance(s) is(are) measured using the above-described analysis chip of the present invention.

In the method of stirring a solution according to the present invention, with the analysis chips, for example, shown in Figs. 2, 3, 11 and 12, the particles 5 move in the upper portion of the separator 4, and are separated from the carrier surface on which the selective binding substance(s) is(are) immobilized by the separator 4. With the analysis chips shown in Figs. 5 to 9, the particles 5 moves on the surface of the vessel 1 below the separator 4, and are separated from the carrier surface on which the selective binding substance(s) is(are) immobilized by the separator 4.

In the method of stirring a solution according to the present invention, as the separator which separates the carrier surface on which the selective binding substance(s) is(are) immobilized and the particles, a structure in the form of a sheet or plate having openings with a size through which the stirrers cannot pass through can be used, which is described above in the description of the modes of the analysis chip of the present invention. Preferably, a lattice-like structure having rectangular openings can be used, and lattice-like mesh is more preferred, and plastic mesh is especially preferred.

In the method of stirring a solution according to the present invention, moving the particles may preferably be carried out by moving the particles utilizing the gravity or magnetic force (in case of magnetic beads), or by applying acceleration to the analysis chip by vibration-shaking or rotation, or combination thereof. Among these, the method wherein the particles are moved by rotating the analysis chip along the plane parallel or substantially parallel to the direction of gravity (the vertical or substantially vertical plane), thereby moving the particles by gravity; and the method wherein the analysis chip is swung describing the figure of 8 in the plane perpendicular or substantially perpendicular to the direction of gravity to move the particles are preferred. The conditions for moving the particles are the same as those described above.

In the present invention, examples of the test substance includes, but are not limited to, nucleic acids to be measured such as genes of pathogenic bacteria and viruses, causative genes of hereditary diseases and parts thereof, various biological components having antigenecity, and antibodies against pathogenic bacteria, viruses and the like.

In the present invention, examples of the solutions containing these test substances include, but are not limited to, body fluids such as blood, serum, plasma, urine, feces, spinal fluid, saliva and various tissue fluids, various foods and drinks, and dilutions thereof.

The nucleic acid employed as the test substance may be a nucleic acid extracted from blood or a cell by a conventional method, which is labeled, or may be one obtained by amplifying the nucleic acid by a nucleic acid-amplification method such as PCR using the nucleic acid as a template. In case of using one amplified by a nucleic acid-amplification method such as PCR using the nucleic acid as a template, the measurement sensitivity can be largely promoted. In cases where an amplified nucleic acid is used as the test substance, by carrying out the amplification in the presence of a nucleotide triphosphate labeled with a fluorescence substance, the amplified nucleic acid can be labeled. In cases where the test substance is an antigen or an antibody, the antigen or the antibody which is a test substance may be directly labeled by a conventional method. A method wherein, after binding the antigen or antibody which is a test substance with the selective binding substance, the carrier is washed, a labeled antibody or antigen which undergoes antigen-antibody reaction with the antigen or antibody is reacted, and the label bound to the carrier is measured, may also be carried out.

The step of bringing the selective binding substance on the carrier into contact with the test substance to allow interaction so as to selectively binding the test substance with the selective binding substance (hybridization reaction) can be carried out in the same manner as in the conventional method. The reaction temperature and time are appropriately selected depending on the size of the nucleic acid to be hybridized, the type of the antigen and/or antibody involved in the immunoreaction and so on and, in case of hybridization of a nucleic acid, the reaction is usually carried out at about 35°C to 70°C for 1 minute to ten and several hours, and in case of immunoreaction, the reaction is usually carried out at room temperature to about 40°C for 1 minute to several hours.

The measurement of the amount of the test substance bound to the carrier through the selective binding substance can be carried out in accordance with a conventional method. For example, it may be attained by a method wherein the analysis chip or the carrier from which the vessel is removed, after the selective binding between the test substance and the selective binding substance, is set in an apparatus such as an existing scanner, and the signal intensity such as fluorescence is measured.

### EXAMPLES

The present invention will now be described in more detail by way of the following Examples. However, the present invention is not limited to the following Examples.

### Example 1

### (Preparation of DNA-immobilized Carrier)

Using the known LIGA (Lithographie Galvanoformung Abformung) process, a mold for injection molding was prepared, and a carrier made of polymethyl methacrylate (PMMA) as the carrier 2 shown in Fig. 16 was obtained. The average molecular weight of the PMMA used in this Example was 50,000, and the PMMA contained carbon black (#3050B produced by MITSUBISHI CHEMICAL) in an amount of 1 wt%, so that the carrier was black. The spectral reflectance and the spectral transmission of this black carrier were measured. As a result, the spectral reflectance was not more than 5% at any wavelength in the visible light range (wavelength of 400 nm to 800 nm), and the transmission was not more than 0.5% at wavelengths in the same range. None of the spectral reflectance and spectral transmission had a particular spectral pattern (such as a peak) in the visible light range, and the spectrum was uniformly flat. As the spectral reflectance, the spectral reflectance taking the regular reflection light from the carrier was measured by an apparatus (CM-2002 produced by MINOLTA CAMERA) carrying an illumination-light receiving optical system in accordance with Condition C of JIS Z 8722.

As for the shape of the carrier, the size was 76 mm long, 26 mm across and 1 mm thick, and the surface was flat except for the central portion thereof. In the center of the carrier, a recess sizing 13 mm square and 0.15 mm deep was formed, and 256 (16 x 16) protrusions 7 having a diameter of 0.15 mm and a height of 0.15 mm, of which surface a selective binding substance was to be bound. The difference between the height of the upper surface of the protrusions in the irregular portion (mean value of the height of the 256 protrusions) and the height of the flat portion was measured, which was not more than 3 µm. The dispersion in height of the upper surfaces of the 256 protrusions (the difference between the largest height of the upper surface of a protrusion and the smallest height of the upper surface of a protrusion), and the difference between the mean value of the height of the upper surfaces of the protrusions and the height of the upper surface of the flat portion were measured. As a result, both of them were not more than 3 µm.

### (Immobilization of Probe DNA)

The above-described PMMA carrier was immersed in aqueous 10N sodium hydroxide solution at 70°C for 12 hours. The carrier was then washed sequentially with pure water, aqueous 0.1N HCl solution and pure water to generate carboxylic groups on the carrier surface.

The DNA (60 bases, 5'-end aminated) having a base sequence shown in SEQ ID NO: 1 was synthesized. The 5'-end of this DNA was aminated.

This DNA was dissolved in pure water to a concentration of 0.3 nmol/µL to obtain a stock solution. When spotting the solution on the carrier, the solution was 10-fold diluted with PBS (8 g of NaCl, 2.9 g of Na₂HPO₄·12H₂O, 0.2 g of KCl and 0.2 g of KH₂PO₄ were dissolved in pure water and then pure water was added to 1L, to which hydrochloric acid for adjusting pH was added, pH5.5) to attain a final concentration of probe DNA to 0.03 mnol/µL, to which 1-ethyl-3-(3-dimethylaminopropyl)carbodiimide (EDC) was added to a final concentration of 50 mg/mL in order to condense the carboxylic acid on the carrier surface with the amino group at the end of the probe. The mixed solution was spotted on all of the upper surfaces of the above-described protrusions on which the carboxylic groups were generated using an arrayer (Gene Stamp-II produced by NIPPON LASER & ELECTRONICS LAB). Then the carrier was placed in a sealed plastic vessel and incubated under conditions of 37°C, 100% humidity for about 20 hours. Finally, the carrier was washed with pure water, and centrifuged and dried by a spin dryer. This reaction scheme is shown in Fig. 20.

### (Separator)

As the separator outer frame 11, a stainless steel plate having a thickness of 0.2 mm was worked to the shape shown in Fig. 13(a), and a polypropylene mesh cloth (SEMITEC; opening 250 µm, wire diameter 103 µm) cut to 20 mm square as the separator member 12 was fixed to the separator outer frame 11 with PDMS polymer (DOW CORNING TORAY) to provide the separator 4 shown in Fig. 13(c). This separator was adhered to the carrier with the above-described PDMS polymer. The adhesion conditions were 42°C for 2 hours.

### (Vessel for Retaining Liquid)

The vessel 1 having 4 through holes 3 shown in Fig. 16 was prepared by injection molding. The depth H2 of the recesses was 1.8 mm. After sonically washing the vessel immersing the vessel in a cleaning agent (produced by CLEAN ACE (ASONE Catalogue, produce number:4-078-01), 25-fold diluted solution) for 5 minutes, the vessel was well rinsed with reverse osmosis water (RO water) and dried by air blowing.

### (Assemblage of Chip)

The separator was placed such that it covered the recesses in the center of the carrier on which the probe DNA was immobilized, and the washed vessel 1 was arranged to cover the separator, followed by adhering the carrier 2 and the vessel 1 with PDMS polymer (Fig. 16). The adhesion conditions were 42°C for 2 hours.

### (Preparation of Particles and Sealing)

Commercially available zirconia particles (produced by TORAY INDUSTREIS, INC.) having a surface roughness of 20 nm and an average particle size of 500 µm were polished in water using a silicon carbide abrasive (particle size #20) by a cyclone barrel polishing machine for 1 hour, washed with water and dried. The surface roughness Ra of the obtained particles was 165 nm. The measurement of the surface roughness of the particles was carried out by depositing Au on the surface by vacuum deposition, and then measuring the surface roughness Ra (nm) by a scanning electron microscope (produced by ELIONIX, type ESA-2000). The surface roughness was measured at an observation magnification x10,000 and setting the cut-off value to 0, and the mean value of the results for arbitrary 10 particles was calculated. The particle size of the particles was measured by taking photographs of the images of arbitrary 100 or more particles observed with a stereoscopic microscope at a magnification of x50 to x150, and their circle equivalent diameters were obtained by an image processing analysis software (produced by MITANI COFRPORATION, Win Roof), followed by calculating the mean value thereof to obtain the average particle size. Thereafter, the particles were immersed in an ethanol solution and ultrasonic cleaning was carried out for 5 minutes. The same washing was repeated another twice. An appropriate amount of the particles was sealed in the space 6 on the separator from the through hole 3 in the vessel 1. The appropriate amount was the amount with which about the half of the upper surface of the separator 4 was completely buried, which amount had been known to be sufficient.

### (Preparation of Test Substance DNA)

As a test substance DNA, a DNA (968 bases) having the base sequence shown in SEQ ID NO: 4 which was able to hybridize with the above-described probe DNA immobilized on the DNA-immobilizing carrier was used. The preparation method thereof is now described.

The DNA having the base sequence shown in SEQ ID NO: 2 and the DNA having the base sequence shown in SEQ ID NO: 3 were synthesized. These were dissolved in pure water to a concentration of 100 µM. Then pKF3 plasmid DNA (TAKARA BIO) (the DNA having the base sequence shown in SEQ ID NO: 5, 2264 bases) was provided. Using this as a template and the DNAs having the base sequences shown in SEQ ID NO: 2 and SEQ ID NO: 3, respectively, as primers, amplification was performed by PCR (Polymerase Chain Reaction).

The conditions of PCR were as follows. That is, 2 µL of ExTaq, 40 µL of 10 × ExBuffer, 32 µL of dNTP Mix (produced by TAKARA BIO), 2 µL of solution of the DNA having the base sequence shown in SEQ ID NO: 2, 2 µL of solution of the DNA having the base sequence shown in SEQ ID NO: 3, and 0.2 µL of template (the DNA having the base sequence shown in SEQ ID NO: 5) were mixed, and pure water was added thereto to a total volume of 400 µL. The mixture was divided into 4 microtubes, and PCR was performed using a thermal cycler. The product was purified by ethanol precipitation and dissolved in 40 µL of pure water. An aliquot of the solution after the PCR was sampled and subjected to electrophoresis. As a result, the length of the amplified DNA was about 960 bases, so that it was confirmed that the DNA (968 bases) having the base sequence shown in SEQ ID NO: 4 was amplified.

Then a random primer (produced by TAKARA BIO) having a size of 9 bases was dissolved to a concentration of 6 mg/ml, and 2 µL thereof was added to the above-described solution of DNA purified after PCR. This solution was heated to 100°C and then quickly cooled on ice. To the resultant, 5 µL of a buffer attached to Klenow Fragment (produced by TAKARA BIO) and 2.5 µL of a dNTP mixture (the concentration of each of dATP, dTTP and dGTP was 2.5mM, and the concentration of dCTP was 400 µM). Further, 2 µL of Cy3-dCTP (produced by GE HEALTH CARE BIOSCIENCES) was added. To this solution, 10U of Klenow Fragment was added, and the mixture was incubated at 37°C for 20 hours to obtain a test substance DNA labeled with Cy3. Since a random primer was used in the labeling, the size of the test substance DNA was dispersed. The longest test substance DNA had the base sequence (968 bases) shown in SEQ ID NO: 4. The solution of the test substance DNA was sampled and subjected to electrophoresis. As a result, the band having the highest intensity appeared at the position corresponding to 960 bases, and slight smear was observed at the regions corresponding to the base length shorter than this. This was purified by ethanol precipitation and dried.

This labeled test substance DNA was dissolved in 400 µL of 1 wt% BSA (bovine serum albumin), 5 × SSC (5 × SSC means 20 × SSC (produced by SIGMA) 4-fold diluted with pure water. Similarly, the solution obtained by 2-fold diluting 20 × SSC with pure water is designated as 10 × SSC, and the solution obtained by 100-fold diluting 20 × SSC with pure water is designated as 0.2 × SSC), 0.1 wt % SDS (sodium dodecyl sulfate) and 0.01 wt% salmon sperm DNA (all of the concentrations are final concentrations) to obtain a stock solution for hybridization.

In the following Examples and Comparative Examples, as the test substance DNA solution for hybridization, a solution prepared by 200-fold diluting the thus obtained stock solution for hybridization with a solution of 1 wt% BSA, 5 × SSC, 0.01 wt% salmon sperm DNA and 0.1 wt % SDS (all of the concentrations are final concentrations). The concentration of the test substance DNA in this solution was measured, which was 3 ng/µL.

### (Hybridization)

Using a micropipette, from the through hole 3 in the vessel 1, the thus prepared test substance DNA solution was injected into the space (space) 6 enclosed by the vessel 1 and the carrier such that the solution did not overflow from the through hole 3. At this time, the solution was able to be easily injected without introducing bubbles. Using a silicone tape (ASONE) as a sealing material, the 4 through holes 3 were sealed. A hybridization chamber (Takara Hybridization chamber (TAKARA BIO) was tightly contacted with a sheet-shaking base (TOKYO RIKAKIKAI) to fix the chamber, and the analysis chip was set in the hybridization chamber. At this time, 15 µL each of ultrapure water was dropped in each of the recesses located at the both ends of the portion at which the analysis chip was to be set. After covering the hybridization chamber with a lid and fixing the lid with 6 fixing screws, the hybridization chamber was mounted and fixed on a shaker placed in a thermostat chamber (FMS-1000, produced by TOKYO RIKAKIKAI). The front face of the thermostat chamber was shaded with an aluminum foil, and the analysis chip was incubated at 42°C for 16 hours with rotation shaking at 250 rpm. After the incubation, the analysis chip was taken out of the hybridization chamber, and the vessel 1 was removed to disassemble the analysis chip. After peeling off the PDMS polymer (adhesive), the particles 5 and the separator 4 were removed to leave the carrier 2 alone, and this carrier 2 was washed and dried.

### (Measurements and Evaluations)

The thus treated carrier was set on a scanner (GenePix 4000B, produced by AXON INSTRUMENTS) for DNA chips, the fluorescence intensity was measured setting the laser output to 33%, the voltage of the photomultiplier to 450, the resolution (pixel size) in data scanning to 10 µm. Based on the thus obtained scanned image, 3 items, that is, the fluorescence intensity (Evaluation A), existence of defects in the spot (Evaluation B), dispersion among spots were evaluated as described below. The results are shown in Table 1. A partial cross-sectional view schematically showing the analysis chip used in Example is shown in Fig. 21 (a).

### <Evaluation A: Fluorescence Intensity>

The fluorescence intensities of the 256 spots (surface of protrusions) on the carrier on which the probe DNA was immobilized were measured, and the hybridization reaction between the test DNA and the probe DNA was evaluated. The fluorescence intensity was the mean value of the fluorescence intensities of all spots. The fluorescence intensity of each spot was the median of the fluorescence intensities of all pixels (the pixel size was 10 µm). The detection limit (upper limit) of the scanner was 50,000. When the fluorescence intensity was 35,000 to 50,000, the fluorescence intensity was evaluated to be large and sufficient and indicated as "+", when the fluorescence intensity was 35,000 to 15,000, the fluorescence intensity was evaluated to be low and indicated as "-", and when the fluorescence intensity was not more than 15,000, the fluorescence intensity was evaluated to be insufficient and indicated as "--".

### <Evaluation B: Existence of Defects in Spot >

In the obtained scanned image, for each of the 256 spots, existence of defects and degree thereof were visually examined to evaluate the influence on the probe DNA-immobilized surface by the particles which move during the stirring of the hybridization solution. Under the set scanning resolution, when no defect was detected in all of the 256 spots, the result was evaluated as "+" (no defect), when a defect was detected even in only one spot among the 256 spots and the area of the defect occupied by the defect in the area of each spot was less than half, the result was evaluated as "-", and when the area of the defect occupied by the defect in the area of each spot was not less than half, the result was evaluated as "--". Although when the area occupied by the defect in one spot is less than half, the fluorescence intensity is not influenced, when it is not less than half, the fluorescence intensity value is influenced.

### <Evaluation C: Dispersion among Spots>

By examining the dispersion of the fluorescence intensities among the 256 spots, the stirring effect of the hybridization solution was evaluated. The fluorescence intensities of the spots in which no defect was detected in Evaluation B were compared. When the difference in the fluorescence intensity of a spot from the mean fluorescence intensity was less than 10%, the result was evaluated as "+" (no spot-to-spot dispersion), and when there was even one spot whose difference in fluorescence intensity was not less than 10%, the result was evaluated as "-" (spot-to-spot dispersion exists).

As a result of the above-described Evaluations A to C, since the obtained fluorescence intensity in Evaluation A was "+", and since in Evaluation C, no spot-to-spot dispersion was observed ("+"), it was proved that sufficient hybridization reaction was attained by sufficient stirring. In Evaluation B, since no defect was detected in all of the 256 spots ("+"), it was proved that there was no influence on the immobilization surface of the probe DNA by the particles.

### Comparative Example 1

The same experiments and the same measurements and evaluations were carried out as in Example 1 except that the separator 4 was not provided. The results are shown in Table 1. A partial cross-sectional view schematically showing the analysis chip used in Comparative Example is shown in Fig. 21(b).

In Evaluation A, the fluorescence intensity was "-", and was about 50% of that in Example 1. In Evaluation B, there were many spots in which the area of the defect occupies not less than half of the area of each spot ("--"). This is presumably because that since the separator was not provided, the particles contacted the probe DNA-immobilization surface and damaged the probe DNA. In Evaluation C, among the spots in which no defect was detected, spot-to-spot dispersion was not observed ("+").

### Comparative Example 2

The same experiments and the same measurements and evaluations were carried out as in Example 1 except that the separator 4 was not provided, the average particle size of the particles was changed from 500 µm to 197 µm, and that the depth (H2 in Fig. 16) of the vessel 1 retaining the solution was changed from 1.8 mm to 0.13 mm. The results are shown in Table 1. A partial cross-sectional view schematically showing the analysis chip used in this Comparative Example is shown in Fig. 21 (c).

In Evaluation A, the fluorescence intensity was "-", and was about 70% of that in Example 1. This is presumably because that since the particle size was smaller than in Example 1, the stirring force was insufficient. In Evaluation C, spot-to-spot dispersion was not observed ("+"). In Evaluation B, no defect in the spots was detected ("+").

### Example 2

Experiments were carried out when the carrier was a flat PMMA carrier having no irregular portions. More particularly, the same experiments and the same measurements and evaluations were carried out using an analysis chip as in Example 1 except that the carrier 2 shown in Fig. 16 was flat and did not have protrusions 7 on which the selective binding substance was to be immobilized. The location at which the probe was immobilized was the center of the carrier similar to Example 1 wherein the protrusions were formed, and the separator and vessel were arranged at the center of the carrier as in Example 1. The results are shown in Table 1. A partial cross-sectional view schematically showing the analysis chip used in this Example 2 is shown in Fig. 21 (d).

In Evaluation A, the fluorescence intensity was "+" as in Example 1. In Evaluation C, since no spot-to-spot dispersion was observed ("+"), it was proved that sufficient hybridization reaction was attained by sufficient stirring. In Evaluation B, no defect was detected in all of the 256 spots ("+"), it was proved that there was no influence on the immobilization surface of the probe DNA by the particles.

### Example 3

The same experiments and the same measurements and evaluations were carried out using an analysis chip as in Example 1 except that a flat carrier was used as in Example 2; the surface in the carrier on which the probe DNA was immobilized was faced downward and an analysis chip covered with a vessel 1 (having no through holes) from the lower side of the carrier, which vessel retained the solution was used and the particles were moved on the surface of this vessel; the average particle size of the particles was changed from 500 µm to 197 µm; and that the depth (H2 in Fig. 16) of the vessel 1 retaining the solution was changed from 1.8 mm to 0.23 mm. The results are shown in Table 1. A partial cross-sectional view schematically showing the analysis chip used in this Example 3 is shown in Fig. 21 (e).

In Evaluation A, the fluorescence intensity was "+" as in Example 1. This is thought to be because that although the particles size is smaller than in Example 1, the particles were able to freely move since the carrier surface did not have an irregular structure. In Evaluation C, no spot-to-spot dispersion was observed ("+"), and in Evaluation B, no defect in the spots was detected ("+").

### Comparative Example 3

The same experiments and the same measurements and evaluations were carried out using an analysis chip as in Example 1 except that the separator was not provided and a flat carrier was used as in Example 2. The results are shown in Table 1. A partial cross-sectional view schematically showing the analysis chip used in this Comparative Example 3 is shown in Fig. 21(f).

In Evaluation A, the fluorescence intensity was "-", and was about 50% of that in Example 2. In Evaluation B, many spots in which the area of the defect occupies not less than half of the area of each spot were detected ("--"). This is thought to be because that since the separator was not provided, the particles contacted the probe DNA-immobilization surface and damaged the probe DNA. In Evaluation C, among the spots in which no defect was detected, spot-to-spot dispersion was not observed ("+").

### Comparative Example 4

The same experiments and the same measurements and evaluations were carried out using an analysis chip as in Example 1 except that the separator was not provided; a flat carrier was used as in Example 2; the average particle size of the particles was changed from 500 µm to 197 µm; and that the depth (H2 in Fig. 16) of the vessel 1 retaining the solution was changed from 1.8 mm to 0.13 mm. The results are shown in Table 2. A partial cross-sectional view schematically showing the analysis chip used in this Comparative Example 3 is shown in Fig. 22(a).

In Evaluation A, the fluorescence intensity was "--". In Evaluation B, many spots in which the area of the defect occupies not less than half of the area of each spot were detected ("--"). This is thought to be because that since the separator was not provided, the particles contacted the probe DNA-immobilization surface and damaged the probe DNA. In Evaluation C, among the spots in which no defect was detected, spot-to-spot dispersion was not observed ("+").

### Comparative Example 5

The same experiments and the same measurements and evaluations were carried out using an analysis chip as in Example 1 except that the separator was not provided; a flat carrier was used as in Example 2; the surface in the carrier on which the probe DNA was immobilized was faced downward, an analysis chip covered with a vessel 1 (having no through holes) from the lower side of the carrier, which vessel retained the solution was used and the particles were moved on the surface of this vessel; the average particle size of the particles was changed from 500 µm to 197 µm; and that the depth (H2 in Fig. 16) of the vessel 1 retaining the solution was changed from 1.8 mm to 0.13 mm. The results are shown in Table 2. A partial cross-sectional view schematically showing the analysis chip used in this Comparative Example 5 is shown in Fig. 22(b).

In Evaluation A, the fluorescence intensity was "-". In Evaluation B, several spots in which the area of the defect occupied less than half of the area of each spot were detected ("-"). This is thought to be because that since the separator was not provided, the particles contacted the probe DNA-immobilization surface and damaged the probe DNA. In Evaluation C, among the spots in which no defect was detected, spot-to-spot dispersion was not observed ("+").

### Comparative Example 6

The same experiments and the same measurements and evaluations were carried out using an analysis chip as in Example 1 except that the separator was not provided; a flat carrier was used as in Example 2; the surface in the carrier on which the probe DNA was immobilized was faced downward, an analysis chip covered with a vessel 1 (having no through holes) from the lower side of the carrier, which vessel retained the solution was used and the particles were moved on the surface of this vessel; the average particle size of the particles was changed from 500 µm to 197 µm; and that the depth (H2 in Fig. 16) of the vessel 1 retaining the solution was changed from 1.8 mm to 0.43 mm. The results are shown in Table 2. A partial cross-sectional view schematically showing the analysis chip used in this Comparative Example 6 is shown in Fig. 22(c).

In Evaluation A, the fluorescence intensity was "--". In Evaluation B, no defect was detected in the spots ("+"), but in Evaluation C, spot-to-spot dispersion was observed ("--"). This is thought to be because that although the particles did not contact the probe-immobilizing surface since a sufficient clearance was provided on the probe DNA-immobilizing surface, the particles size relative to the reaction space was small, so that the stirring of the solution was insufficient.

### Comparative Example 7

The same experiments and the same measurements and evaluations were carried out using an analysis chip as in Example 1 except that the separator was not provided; a flat carrier was used as in Example 2; the surface in the carrier on which the probe DNA was immobilized was faced downward, an analysis chip covered with a vessel 1 (having no through holes) from the lower side of the carrier, which vessel retained the solution was used and the particles were moved on the surface of this vessel. The results are shown in Table 2. A partial cross-sectional view schematically showing the analysis chip used in this Comparative Example 7 is shown in Fig. 22(d).

In Evaluation A, the fluorescence intensity was "+". In Evaluation B, several spots in which the area of the defect occupied less than half of the area of each spot were detected ("-"). This is thought to be because that since the separator was not provided, the particles contacted the probe DNA-immobilization surface and damaged the probe DNA. In Evaluation C, spot-to-spot dispersion was not observed ("+").

### Comparative Example 8

The same experiments and the same measurements and evaluations were carried out using an analysis chip as in Example 1 except that the separator was not provided; a flat carrier was used as in Example 2; the surface in the carrier on which the probe DNA was immobilized was faced downward, an analysis chip covered with a vessel 1 (having no through holes) from the lower side of the carrier, which vessel retained the solution was used and the particles were moved on the surface of this vessel; and that the depth (H2 in Fig. 16) of the vessel 1 retaining the solution was changed from 1.8 mm to 3.6 mm. The results are shown in Table 2. A partial cross-sectional view schematically showing the analysis chip used in this Comparative Example 8 is shown in Fig. 22(e).

In Evaluation A, the fluorescence intensity was "--". In Evaluation B, no defect was detected in the spots ("+"), but in Evaluation C, spot-to-spot dispersion was observed ("-"). This is thought to be because that although the particles did not contact the probe-immobilizing surface since a sufficient clearance was provided on the probe DNA-immobilizing surface, the particles size relative to the reaction space was small, so that the stirring of the solution was insufficient.

[Table 1]

[Table 2]

### INDUSTRIAL APPLICABILITY

The present invention enables to promote the reaction efficiency and attaining a high sensitive analysis in the analysis chip in which a test substance(s) and an immobilized selective binding substance(s) are reacted while stirring the test substance solution using particles, and is useful in the fields of pharmaceuticals and medicine, foods, environment and the like.

### DESCRIPTION OF SYMBOLS

1 vessel
2 carrier
3 through hole
3A liquid surface-halting chamber
4 separator
5 particles
6 space (space)
6A well structure
7 protrusions immobilizing the selective binding substances on the surface thereof
8 selective binding substance
9 protrusions on the vessel surface
10 sealing member
11 separator outer frame
12 separator member
13 protrusions on carrier surface
14 flat portion
15 carrier
16 jig
17 spring for abutting microarray to jig
18 objective lens
19 laser excitation light
20 DNA
R1 immobilization region on which selective binding substances are immobilized
L1 protrusion pitch
H1 protrusion height
H2 depth of recesses in vessel

[SEQUENCE LISTING]

## Claims

1. An analysis chip comprising:
a carrier having a surface on which a selective binding substance(s) is(are) immobilized;
a vessel holding a solution containing a test substance(s) which react(s) with said selective binding substance(s); and
particles for stirring said solution, said particles being sealed within a space formed between said carrier and said vessel;
wherein a separator which allows passage therethrough of said solution containing said test substance(s) but does not allow passage therethrough of said particles is arranged in said space so as to separate said surface of said carrier on which said selective binding substance(s) is(are) immobilized and said particles.

2. The analysis chip according to claim 1, wherein said separator is a structure in the form of a sheet or plate having openings.

3. The analysis chip according to claim 1, wherein said separator is a lattice-like structure.

4. The analysis chip according to claim 1, wherein said separator is a mesh.

5. A method of analyzing a test substance(s), said method comprising the steps of:
contacting said test substance(s) with said analysis chip according to any one of claims 1 to 4;
moving said particles to selectively bind said test substance(s) with said selective binding substance(s) on said carrier; and
measuring amount of said test substance(s) bound to said carrier through said selective binding substance(s).

6. A method of stirring a solution, said method comprising contacting the solution containing a test substance(s) which react(s) with a selective binding substance(s), with the selective binding substance(s) immobilized on a carrier surface; and stirring said solution with particles; wherein the particles are separated from said carrier surface on which said selective binding substance(s) is(are) immobilized, by a separator which allows passage therethrough of said solution containing said test substance(s) but does not allow passage therethrough of said particles.

7. The method of stirring a solution, according to claim 6, wherein said separator is a structure in the form of a sheet or plate having openings.

8. The method of stirring a solution, according to claim 6, wherein said separator is a lattice-like structure.

9. The method of stirring a solution, according to claim 6, wherein said separator is a mesh.
**Table 1**
| | Example 1 | Comparative Example 1 | Comparative Example 2 | Example 2 | Example 3 | Comparative Example 3 |
|---|---|---|---|---|---|---|
| Shape of Carrier | irregular | irregular | irregular | flat | flat | flat |
| Existence of Separator | existed | no | no | existed | existed | no |
| Average Particle Size of Particles (µm) | 500 | 500 | 197 | 500 | 197 | 500 |
| Depth of Recesses in Vessel H2 (mm) | 1.8 | 1.8 | 0.13 | 1.8 | 0.13 | 1.8 |
| Evaluation A Evaluation of Fluorescence Intensity (values in parentheses indicate mean value of fluorescence intensities of 256 spots) | + (48000) | - ( 25000 ) | - ( 33000 ) | + ( 47000 ) | + ( 42000 ) | - ( 24500 ) |
| Evaluation B Existence of Defect in Spot | + | -- | + | + | + | -- |
| Evaluation C spot-to-spot dispersion | + | + | + | + | + | + |
**Table 2**
| | Comparative Example 4 | Comparative Example 5 | Comparative Example 6 | Comparative Example 7 | Comparative Example 8 |
|---|---|---|---|---|---|
| Shape of Carrier | flat | flat | flat | flat | flat |
| Existence of Separator | no | no | no | no | no |
| Average Particle Size of Particles (µm) | 197 | 197 | 197 | 500 | 500 |
| Depth of Recesses in Vessel H2 (mm) | 0.13 | 0.13 | 0.43 | 1.8 | 3.6 |
| Evaluation A Evaluation of Fluorescence Intensity (values in parentheses indicate mean value of fluorescence intensities of 256 spots) | -- (14000) | - (30000) | -- (14000) | + (45000) | -- (15000) |
| Evaluation B Existence of Defect in Spot | -- | - | + | - | + |
| Evaluation C spot-to-spot dispersion | + | + | - | + | - |
